# EUROPEAN PATENT SPECIFICATION

(11) **EP 2 142 199 B1**
(45) Date of publication and mention of the grant of the patent: **05.08.2015**
(21) Application number: 08734966.8
(22) Date of filing: 02.04.2008
(51) Int. Cl.: A61K 36/06, A61K 36/10, A61K 36/11, A61K 8/97, A61K 8/99, A61K 35/60, A61K 35/68, A61K 35/74, A61K 41/00, A61Q 19/08, A61P 39/06

(54) **A PROCESS TO ACTIVATE EXTRACTS DERIVED FROM LIVING ORGANISMS USING LONG WAVE UV IRRADIATION AND COMPOSITIONS COMPRISING SAID ACTIVATED EXTRACT**
VERFAHREN ZUR AKTIVIERUNG VON EXTRAKTEN AUS LEBENDEN ORGANISMEN MIT LANGWELLEN-UV-BESTRAHLUNG UND ZUSAMMENSETZUNGEN MIT DIESEN AKTIVIERTEN EXTRAKTEN
PROCÉDÉ D'ACTIVATION D'EXTRAITS DÉRIVÉS D'ORGANISMES VIVANTS UTILISANT UN RAYONNEMENT DE GRANDE LONGUEUR D'ONDE ET COMPOSITIONS COMPRENANT LEDIT EXTRAIT ACTIVÉ

(30) Priority: 03.04.2007 EP 07006989
(43) Date of publication of application: 13.01.2010
(73) Proprietor: Medizinische Universität Wien, 1090 Wien (AT)
(72) Inventor: GRUBER, Florian, A-1050 Wien (AT); OSKOLKOVA, Olga, 1040 Wien (AT); TSCHACHLER, Erwin, 1170 Wien (AT); BOCHKOV, Valery, 1100 Wien (AT)
(74) Representative: Vossius & Partner Patentanwälte Rechtsanwälte mbB
(86) International application number: PCT/EP2008/002622
(87) International publication number: WO 2008/119556

(56) References cited:
- WO-A-00/59303
- GRUBER F ET AL: "Photo-oxidation of the membrane phospholipid PAPC induces heme oxygenase 1 expression in skin cells" JOURNAL OF INVESTIGATIVE DERMATOLOGY, NATURE PUBLISHING GROUP, GB, vol. 125, no. 3, Suppl. S, 1 September 2005 (2005-09-01), page A55, 321, XP009118493 ISSN: 0022-202X
- GRIMSLEY N H ET AL: "FATTY-ACID COMPOSITION OF MUTANTS OF THE MOSS PHYSCOMITRELLA-PATENS" PHYTOCHEMISTRY (OXFORD), vol. 20, no. 7, 1981, pages 1519-1524, XP002533758 ISSN: 0031-9422
- GRUBER F ET AL: "Photooxidation generates biologically active phospholipids that induce heme oxygenase-1 in skin cells" JOURNAL OF BIOLOGICAL CHEMISTRY 20070608 US, vol. 282, no. 23, 8 June 2007 (2007-06-08), pages 16934-16941, XP002533759 ISSN: 0021-9258 1083-351X

## Description

The present invention relates to a method for producing an extract which is capable of inducing an antioxidant stress response comprising the step of treating an extract from organic material with UV-A1 light, wherein a fluency of at least 1 J/cm² to 200 J/cm² of said UV light is applied, wherein said extract from organic material is derived from a bryophyte or algae capable of synthesizing polyunsaturated fatty acids comprising at least 4 double bonds and at least 20 C-atoms, and wherein said extract from organic material is a lipid extract comprising at least 0.1 (v/v) % and at the utmost 50 (v/v) % 1-palmitoyl-2-arachidonyl-sn-glycero-3-phosphorylcholine (PAPC). Furthermore, an extract capable of inducing an antioxidant stress response produced in accordance with the inventive methods, in particular an extract capable of inducing an antioxidant stress response for the preparation of a cosmetic composition is disclosed. The present invention also relates therefore to a cosmetic composition comprising the inventive extract obtainable by the method provided herein. It is also disclosed herein that the extract obtained/obtainable by the method of the invention may also be used in a pharmaceutical composition. The pharmaceutical composition as disclosed herein may, inter alia, be of value in the treatment, prevention and/or amelioration of disorders/diseases related to antioxidant stress as well as disorders like skin cancer, clinical sunburn, certain inflammatory diseases, psoriasis and eczema.

The role of oxidized lipids in enhancing the development of atherosclerotic lesions has been extensively investigated in past years. Oxidized lipids which are present in minimally modified-low density lipoproteins (MM-LDL) contribute to the accumulation of monocyte/macrophages within the vessel wall by activating endothelial cells to express monocyte-specific adhesion molecules and chemo attractant factors. This accumulation is characteristic of the fatty streak which is the precursor of the atherosclerotic lesion (Watson, JBC 274 (35), 24787-24798 (1999)). In view of this, oxidized lipids *in general* are regarded as something "bad", because they have been discovered and they are formed in situations like inflammation and cellular stress.

In order to counteract negative effects of oxidized lipids in cells, particularly in skin cells, various strategies were followed in the art. For example, skin care products are employed for achieving an antioxidant, anti-aging or protective effect. Mainly, miscellaneous active compounds are employed in approaches avoiding oxidation (like vitamin c formulations, spin trap approaches) or applying exfoliants, such as Alpha Hydroxy Acid (AHA) and Beta Hydroxy Acid (BHA) formulations, that additionally induce skin repair amongst others.
However, use of the aforementioned substances may have negative side effects, as shown in studies performed by the Cosmetic, Toiletry and Fragrance Association (CTFA) and FDA on the safety AHAs in cosmetic products. These studies which were reported at a December 1996 meeting of the trade group's Cosmetic Ingredient Review Panel (CIR) demonstrated an increased skin sensitivity to UV radiation due to topical application of AHAs. The FDA suggested to use a sunscreen and limit sun exposure while using a product containing AHAs and for a week afterwards. Moreover, it is known in the art that AHAs can produce reactions such as skin irritations, peeling, redness, and flaking.

The Alpha Hydroxy Acids include glycolic acid, lactic acid, tartaric acid, malic acid, and citric acids. Alpha Hydroxy Acids are primarily an exfoliant but also stimulate skin repair, increase collagen production, increase skin thickness, improve elasticity, are utilized as an effective acne treatment, improve skin texture, improve skin tone and decrease enlarged pores. The same applies to BHA. Other actives include vitamin A, spin traps and vitamin C. Retinol skin care and the vitamin A derivatives stimulate skin repair, increases collagen production, increases skin thickness, whereas spin trap is described as an intelligent antioxidant that transforms skin damaging free radical activity into productive regenerative activity by trapping and detoxifying free radicals. As with other antioxidants, spin trap is particularly noted for its skin protective properties, although spin trap has been touted as the next generation of anti-aging treatments. In its stable forms, Vitamin C skin care products are clinically proven to be an effective skin rejuvenation treatment. These actives all either act directly as antioxidants (Vitamins and spin traps) or have profound biological effects on skin cells that coincide with sun sensitivity (AHA and Retinoic Acid).

However, the methods for skin protection are known in the art and have also undesirable effects, like increased sun sensitivity and eventually skin irritation and stinging.

Aged tissue, as aged skin, shows decreased synthesis of biomolecules that degrade damaged material that accumulates in the cells. This material includes advanced glycation end products (AGEs), and other potentially dangerous substances. Aged tissues also show decreased synthesis of biomolecules that prevent the oxidative damage before it can happen, most prominent glutathione (GSH).

Inflammatory skin diseases such as atopic dermatitis, scleroderma and graft versus host disease are treated by phototherapy using UVA-1 irradiation (reviewed in Mang, Photodermatol.Photoimmunol.Photomed. 21, 103-108, (2005)). Some of the benefits of UVA-1 phototherapy include a decrease of T-cells by UV-induced apoptosis (Morita, J.Exp.Med. 186, 1763-1768, (1997)) or a decrease in the Th1/Th2 cell ratio in the inflamed tissue (Szegedi, Rheumatology (Oxford) 44, 925-931, (2005)). Also expression of Heme oxygenase 1 (HO-1) is up-regulated as a cellular response to oxidative stress such as induced by UVA irradiation (reviewed in Ryter, Antioxid.Redox.Signal. 4, 625-632(2002))) or during inflammation (reviewed in Ryter, Mol.Cell Biochem. 234-235, 249-263 (2002)). Induction of HO-1 as observed during inflammation and in the early phase of wound repair involves the generation of reactive oxygen species (ROS) (Hanselmann, Biochem.J. 353, 459-466, (2001), Wagener, Blood 102, 521-528, (2003)). In the skin, UVA irradiation generates ROS that modify cellular lipids and proteins and induces gene regulation (Grether-Beck, Biol.Chem. 378, 1231-1236, (1997), Ichihashi, Toxicology 189, 21-39, (2003), Krutmann, J.Dermatol.Sci. 23 Suppl 1, S22-S26, (2000)). In a long term view however, bad effects of UVA-1 irradiation will certainly surpass initial beneficial effects. Therefore, it is highly desirable to find active substances that substitute for the beneficial effects of UVA-1 irradiation without conferring any detrimental effects.

It is known that oxidation products of 1-palmitoyl-2-arachidonoyl-*sn*-glycero-3-phsophorylcholine (PAPC), a common phospholipid, are associated with the enhancement of inflammatory processes. In 1999, Watson identified chemically synthesized and isolated 1-palmitoyl-2-(epoxyisoprostane E2)-sn-glycero-3-phosphorylcholine (PEIPC) as an epoxyisoprostanoid oxidation product present in MM-LDL as well as in autoxidized PAPC (oxPAPC), while other oxidized derivatives of phospholipids comprising a group derived from arachidonic acid, such as 1-palmitoyl-2-(5)oxovaleryl-sn-glycero-3-phosphocholine (POVPC) and 1-palmitoyl-2-glutaryl-sn-glycero-3-phosphocholine (PGPC) had already been identified before (Watson, JBC 274 (35), 24787-24798 (1999)). However, Watson used autoxidized PAPC which comprises a larger number of oxidation products of unknown function compared to UVA-1 treated PAPC. Furthermore, Watson used chemically obtained and isolated PAPC while the present invention provides for a natural source comprising PAPC.

In 2002, Subbanagounder found that chemically produced PEIPC isomers are potent activators of endothelial cells increasing synthesis of interleukin-8 (IL-8) und monocyte chemotactic protein-1 (MCP-1) which are important regulators of atherogenesis and increased in human atherosclerotic lesions (Subbanagounder, JBC 277 (9), 7271-7281 (2002)). Walton used oxPAPC to induce interleukin-8 in aortic endothelial cells by binding to the Toll-like receptor 4 (TLR4) (Walton, JBC 278 (32), 29661-29666 (2003)).
Lipid oxidation products derived from various phospholipid categories, e.g. physophatidylcholine, -ethanolamine, -serine, are used for the inhibition of inflammatory processes, that are caused by bacterial infection. In particular, autoxidized PAPC, respectively one identified oxidation product, POVPC, was used for the inhibition of the activation of TLR4 (Toll-like receptor 4) by LPS (lipopolysaccharid, component of the membrane of Gram-negative bacteria) in endothelial cells, see WO 2004/006910.

The use of oxidized phospholipids such as oxPAPC or epoxyisoprostane-containing phospholipids such as PEIPC for the treatment of disorders such as acute lung injury, sepsis and acute respiratory distress syndrome (ARDS) was described. These disorders are caused by increased lung vascular permeability. By administering oxidized phospholipds, in particular epoxyisoprostane-containing phospholipids, the endothelial cell barrier function was increased, see US 2006/0194765. Li found that Prostaglandin E2 receptor EP2 is activated in endothelial cells and macrophages by oxPAPC and PEIPC either as the phospholipid ester or the released fatty acid. (published online - Circulation Research, DOI: 10.1161/01.RES.0000207394.39249.fc (2006). Oxidized lipids, in particular phospholipids, were used for the treatment and prevention of inflammation. It has also been speculated that a single oxidized hydrocarbon chain such as arachidonic acid metabolites exerts the same antigenicity activity as the oxidized phospholipids, see WO 2004/106486.

Though in the scientific literature and patent applications described above, oxidation products of chemically synthesized and isolated PAPC, such as PEIPC or POVPC, are determined as regulators of inflammatory processes, it was not referred to a putative role of oxidized lipids as inducers of an antioxidant response. Merely synthetic autoxidized PAPC oxidation products were used in the art.

Most of the polyunsaturated fatty acids such as omega-3 and omega-6 fatty acids can be produced in humans while the fatty acids forming the starting material such as α-linolenic acids or linoleic acid have to be supplied by external sources. Even though humans are able to produce polyunsaturated fatty acids (PUFAs), it is generally known that an additional food supply of these fatty acids exerts a beneficial effect on human health.

The following relates to organisms that produce polyunsaturated fatty acids and might therefore serve as sources of PUFAs. PUFAs are found in some gymnosperms and many algae, mosses and ferns (Wolff, Lipids 35, 1-22 (2000), Ackmann, J. Fish. Res. Bd. Can 25, 1603-1620 (1968), Dembitsky, Prog Lipid Research 32 p. 281-356 (1993) and Jamieson Phytochemistry 14, p. 2229-2232 (1975) cited in: Kaewsuwan, JBC 281(31), 21988-21997 (2006); Zhukova, Phytochemistry 39, 351-356 (1995) cited in Girke, Plant Journal, 15 (1), 39-48, (1998)). Yet, it is known that the moss *Physcomitrella patens* contains arachidonic acid and some eicosapentanoic acids (Grimsley, Phytochemistry 20, 1519-1524 (1981), cited in Kaewsuwan, JBC 281(31), 21988-21997 (2006)).

PUFA such as docosahexeanoic acid (DHA) and eicosapentaenoic acid (EPA) may also be found in species of marine bacteria such as *Shewanella* while a eukaryotic marine protist such as *Schizochytrium* contains DHA or docosapentaenoic acid (DPA) (Nichols, Curr opin Biotechnol 10, 240 (1999), Yazawa, Lipids 31, S-297 (1996), Delong, Appl Environ Microbiol 51, 730 (1986), Barclay, J Appl Phycol 6, 123 (1994), cited in: Metz, Science 293, 290-293 (2001)).

Also marine fish oil is a known source for polyunsaturated fatty acids (Jenkins (1990) J Dairy Sci 73, 2940-2951; Buda (1994) PNAS 91, 8234-8238).

As discussed above, Watson (1999; loc. cit.) has taught that oxidized chemically isolated synthetic PAPC is capable of activating endothelial cells due to the expression of adhesion molecules and chemo attractant factors. Watson and colleagues speculated that the particular molecule may be produced by free radical oxidation and that this may be important in the regulation of (pro-)inflammatory processes.

The technical problem underlying the present invention is the provision of means and methods for the preparation of highly active compounds that are useful as antioxidant compositions, in particular in products to be employed on or in the human body.

The technical problem is solved by provision of the embodiments characterized in the claims.

Accordingly, the present invention relates to a method for producing an extract capable of inducing an antioxidant stress response, said method comprising the step of treating an extract with UV-A1 light, wherein a fluency of at least 1 J/cm² to 200 J/cm² of said UV light is applied, wherein said extract from organic material is derived from a bryophyte or algae capable of synthesizing polyunsaturated fatty acids comprising at least 4 double bonds and at least 20 C-atoms, and wherein said extract from organic material is a lipid extract comprising at least 0.1 (v/v) % and at the utmost 50 (v/v) % 1-palmitoyl-2-arachidonyl-sn-glycero-3-phosphorylcholine (PAPC). In particular, the extract as provided herein and obtainable by the methods of this invention is capable of inducing the expression of HO-1 (heme oxygenase 1) or the expression of GCLM (Glutathione Cysteine Ligase Modifier subunit), both reporters for a successful antioxidant stress response (see also appended examples).

As documented in the examples provided herein, it was surprisingly found that extracts from organic material of an bryophyte or algae capable of synthesizing polyunsaturated fatty acids can effectively be activated by use of light with an UV-A1 light content. The (UV) light to be employed in the method of this invention should have at least a proportion of UVA-1. The cell/cellular material or said organism to be employed in context of this invention is a non-mammalian cell/cellular material or non-mammalian organism. It was furthermore surprisingly found that extracts activated in accordance with this invention are much more potent in the capability of inducing (an) antioxidant stress response(s) than pure, isolated and/or chemically produced individual chemical substances, like activated PAPC. This was illustrated, inter alia, by the induction of a key enzyme activated/expressed in cellular responses to tissue injuries and oxidative stress, i.e. heme oxygenase 1.

Oxidized lipids and, particularly, mildly oxidized lipids may not only be associated with enhancing inflammatory processes and subsequent putative detrimental effects, but also with evoking beneficial effects such as inducing an antioxidant response or exerting an antiproliverative activity on tumor cells.

WO 00/59303 shows that autoxidized products of 20 and 22 carbon groups containing polyunsaturated fatty acids, in particular arachidonic acid and docosahexanoic acid, have potent anti-proliverative activity when applied to tumor cells as compared to their unoxidized precursors. However, said patent application does not refer to the use of UV-oxidized phospholipids (as provided herein in form of an activated extract). These UV-oxidized phospholipids (and the corresponding extracts) induce an antioxidant response in particular on skin cells. In WO 00/59303 a positive effect is attributed to certain fractions of oxidized arachidonic acid, but the exact chemical nature of the active compound is not determined.

Gruber (2005) J Invest Dermatol 125, A55 and Gruber (2007) J Biol Chem 282, 16934-16941 disclose that UV-A1 irradiated PAPC induces heme oxygenase (HO)-1 expression.

Ishikawa (1997) describes that 1-palmitoyl-2-isoprostanoyl-*sn*-glycero-3-phosphorylcholine (PIPPC) induces an antioxidant response by stimulating the expression of heme oxygenase 1 (HO-1). Expression of Heme oxygenase 1 is induced by various physical and chemical stresses and Ishikawa suggests that HO-1 may function as an antioxidant in artery cell walls (Ishikawa, J Clin Invest 5, 1209-1216 (1997)). However, Ishikawa uses synthetic autoxidized PAPC in order to induce an antioxidant response in endothelial cells. This is in strong contrast to the present invention, wherein activated extracts, i.e. mildly oxidized extracts, derived from a natural source are to be used for the induction of an antioxidant response in skin cells. Again, it was surprisingly found in context of this invention that the inventive, activated extracts are more potent in their antioxidating effect than individual, isolated, pure substances as, for example, used by Ishikawa (1997).

Accordingly, activated (i.e. mildly oxidized) extracts, as provided herein and derived from (a) non-mammalian organism(s), in particular mosses, are much more potent in inducing an antioxidant stress response than individual isolated (mildly) oxidized lipids comprising a group derived from a PUFA (such as autoxidized or UV-treated PAPC). As documented in the enclosed examples, it could be shown that in particular extracts from natural, non-mammalian sources, in particular from lower plants, for example mosses like *Physcomitrella patens,* which are activated by UV-light in accordance with this invention are more potent in activating positive cellular stress responses than isolated and synthetically/ chemically produced (poly)unsaturated fatty acids which were mildly oxidized or than lipids comprising these fatty acids.

WO 02/41827 describes a method to synthesize an esterified oxidized chemically synthesized phospholipid, wherein the precursor comprises a phospholipid backbone including two fatty acid side chains. In said patent application it is speculated to use said synthesized oxidized lipids in inducing immune tolerance to oxidized LDL, thereby treating atherosclerosis and related disorders.
WO 2006/006161 describes the synthesis of oxidized chemically synthesized phospholipids wherein the compounds have a glycerolic backbone and one or more oxidized moieties attached to the glycerolic backbone. However, WO 2006/006161 does neither propose nor disclose that the synthesized oxidized phospholipids may have any biological effect such as inducing an antioxidant stress response.

To summarize, the above mentioned publications relate to the generation of oxidized chemically synthesized lipids either via autoxidation of synthetic lipids or via synthesis of oxidized lipids. In contrast thereto, the method of the present invention relates to the production and provision of an extract obtainable from organic material derived from a bryophyte or algae capable of synthesizing polyunsaturated fatty acids comprising at least 4 double bonds and at least 20 C-atoms, said extract comprising at least 0.1 (v/v) % and at the utmost 50 (v/v) % PAPC, said extract being treated with UVA-1 light, and thereby generating (mildly) oxidized natural lipids or other activated natural compounds.

One advantage of the present invention is that a natural composition such as an extract derived from organic material derived from a bryophyte or algae capable of synthesizing polyunsaturated fatty acids comprising at least 4 double bonds and at least 20 C-atoms comprising at least 0.1 (v/v) % and at the utmost 50 (v/v) % PAPC is to be treated with UVA-1 light. Thus, a chemical synthesis of an oxidized lipid or the chemical treatment of synthesized lipids in order to obtain oxidized lipids can be avoided. Chemical or synthetic products may have negative effects on human health. Thus, the use of an UVA-1 treated natural extract as obtained/obtainable by the method of the present invention is a major advantage in the cosmetic or medical field.

As a further advantageous aspect of the present method, the extract derived from bryophytes or algae, and most preferred from mosses, like the moss *Physcomitrella patens,* exerts a higher antioxidant response, for example when compared with a pure compound such as PAPC. Thus, treatment of e.g. skin cells will be much more effective using said extract than the pure (oxidized) compounds, like chemically synthesized PAPC.

A further advantage of the method of the invention and the corresponding activated extract (i.e. the extract obtainable by said method) is the enhanced formation of long chain oxidation products from unoxidized precursors after UVA-1 light irradiation compared to a conventional method such as autoxidation. Mainly long-chain oxidation products induce the desired antioxidant response as disclosed in the present invention, whereas short chain oxidation products do not induce such an antioxidant response.

As a further advantageous aspect, the method of the invention allows the use of an oxidized lipid formulation or compound mixture in order to induce an antioxidant response instead of use of the putative harmful irradiation with UVA-1, like it is applied in phototherapy.

The experimental part of this invention, inter alia, documents that an activated extract derived from mosses, in particular from the moss *Physcomitrella patens,* ferns, in particular from the fern *Thelypteris noveboracensis,* or fish induces a strong antioxidant response in skin cells. The "activation" of said extract is obtained by (UV-A1)light as shown in the appended examples. Most preferably, said light is UVA-1 or has a proportion of UVA-1 or comprises UVA-1 light.

In one embodiment, the present invention relates to a method for producing an extract capable of inducing an antioxidant stress response comprising the step of treating a extract from organic material with UV light, said extract being derived from (a) bryophyte cell(s) or algal cell(s) or a bryophyte or algal organism capable of synthesizing (poly)unsaturated fatty acids comprising at least 4 double bonds and at least 20 C-atoms. The extract provided herein and obtainable by the method of this invention may be derived from organic material such as a bryophyte or algal single-or multi-cellular organism capable of producing (poly)unsaturated fatty acid.

Generally, any bryophyte or algal organism can be used as long as it is capable of synthesizing or comprises (a) certain polyunsaturated fatty acid(s) or group derived therefrom, namely (a) PUFA(s) comprising at least 4 double bonds and at least 20 C-atoms. Preferably such a PUFA is arachidonic acid. An organism as employed herein capable of synthesizing a (poly)unsaturated fatty acid or group derived therefrom, may also be capable of synthesizing molecules, in particular lipids, which comprise said groups derived from said (poly) unsaturated fatty acids. Preferably said lipid is PAPC.

In appended example 5 it is shown that extracts derived from olive, corn and pumpkin are not capable of inducing an antioxidant response in accordance with this invention. Neither olive, corn nor pumpkin are known to be capable of synthesizing the particular polyunsaturated fatty acids as described and defined herein, like the polyunsaturated fatty acids comprising at least 4 double bonds (see for example Milosevic, Int J Food Science and Technology 37, 523-526 (2002)).

Examples of organisms, the "extract" provided and described herein may be derived from, are, inter alia, *Spirulina spec., Chlorella spec., Laminaria spec., Laminaria saccharina, Ceratodon purpureus* and *Marchantia polymorpha.* Most preferred is moss (*Physcomitrella patens*) as source for the inventive extract.

The term "bacteria" means prokaryotes comprising the evolutionary domains *Bacteria* and *Archaea.* One example for such a bacterium is *Shewanella sp.*

The term "protists" means single- to few-cellular eukaryotes. Protists are, for example, *Dictyostelium discoideum, Phaeodactylum tricomutum* or *Schizochytrium sp..* US 2004/0203121, US 5518918 and US 5340742 describe the synthesis of PUFAs which are produced by the protists *Thraustochytrium* and *Schizochytrium.* Also US 5407957, US 7163811, US 7252979, US 6977166 and US 6207441 describe methods for producing PUFAs and organisms synthesizing PUFAs, such as dinoflagellates. US 7259006 and US 6812009 describe further sources (such as algae and protists) of PUFAs, in particular of docosahexaenoic acid.

Examples for "fungi" include *Mortierella alpina* and *Phytophtora megaspema.* US 2005/0266538 discloses a process for the synthesis of lipids containing arachidonic acid by culturing a microorganism, in particular *Mortierella.*

Accordingly, also such organisms may be employed in context of this invention to provide for an extract to be activated, i.e. obtainable/obtained by the methods disclosed in this invention.

As mentioned above, particularly marine fish oil is a source for polyunsaturated fatty acids, in particular PUFAs or groups derived therefrom comprising at least 4 double bonds. While fish oil contains on average 2,7(+/-2)% of arachidonic acid, the major fatty acids are docosahexaenoic acid (DHA) and eicosapentaenoic acid (EPA) (Soriguer (1997) Eur J Epidem 13, 451-463). Also commercially available fish oils contain arachidonic acid. For example, one softgel of the fish oil concentrate "Max EPA", Thompson nutritional, contains a natural mixture of marine oils, containing in particular 180 mg of eicosapentaenic acid (EPA) and 120 mg of docosahexaenoic acid (DHA). Such a commercially available fish oil contains 2% arachidonic acid; see Jenkins (1990) J Dairy Sci 73, 2940-2951. It is also known that extracts derived from fish contain about 2% to 3% PAPC; see Buda (1994) PNAS 91, 8234-8238.

The meaning of the term "plant(s)" can readily be deduced by the skilled person. In this context the term "plants" can mean photoautotrophic eukaryotes which comprise algae, e.g. red algae (rhodoplantae) or green algae (chlorophyta), stoneworts (charophyta), bryophytes, ferns and fern allies (pteridophyta), gymnosperms (gymnospermae) and angiosperms (angiospermae).

In the context of this invention the bryophyte or algae to be employed are characterized by a high content of certain PUFAs, i.e., by PUFAs or groups derived therefrom comprising at least 4 double bonds and at least 20 C-atoms. Plants to be used may include mosses like *Physcomitrella patens.*

The extract is derived from a bryophyte or algae. Non-limiting examples of algae are, inter alia, *Spirulina spec., Chlorella spec.* or *Laminaria spec.* Even more preferably, said extract is derived from a bryophyte which is selected from the group consisting of liverworts (Marchantiophyta), such as *Marchantia polymorpha,* hornworts (Anthoceratophyta) and mosses (Bryophyta), such as *Ceratodon purpureus.* More preferably, said extract is derived from a bryophyte (moss) which belongs to the genus *Physcomitrella.* Most preferably, said extract is derived from the moss *Physcomitrella patens.* The meaning of the terms "bryophyte", "algae", and of other taxonomic terms are well known in the art, and can, for example be deduced from Strasburger (2002, Spektrum Akademischer

Verlag). The term "lower plant" particularly refers to non-vascular plants comprising, for example, algae and bryophytes.

Useful, organisms to be employed in context of the present invention are also disclosed in Jamieson (Phytochem 14, 2229-2232 (1975)), Dembitsky (Prog. Lipid Res. 32, 281-356 (1993)), Soriguer (Europ J Epidem 13, 451-463 (1997)) and Buda (PNAS 91, 8234-8238 (1994)), US 7163811 and US 5407957.

Usually, higher plants belonging to the group of Gymnosperms or Angiosperms (but not ferns) show high activities in secondary metabolism (which, for example, leads to the production of essential oils), whereas in ferns and lower plants, like mosses and algae, secondary metabolism is exhibited to a lower extent. Therefore, without being bound by theory, particularly the use of plants exhibiting low secondary metabolism (bryophytes or algae) may result in highly cosmetically and toxicologically acceptable "extracts" due to the absence/low level of undesired secondary metabolites (like some essential oils). It is known in the art that secondary metabolites (like some essential oils) can cause undesired side effects, like allergies, (skin) irritations and/or inflammatory reactions.
Accordingly, it is particularly envisaged herein that the extract to be employed and provided contains a low amount of undesired secondary metabolites. Extracts from bryophytes or algae meet this requirement. Therefore, bryophytes or algae, including mosses, are particular preferred sources for the activated extract of the invention.

The extract employed and provided in the context of the present invention may be derived from transgenic bryophytes or algae defined herein that comprise enzymes related to the biosynthesis of the particular PUFA(s) as described herein, i.e. PUFA(s) comprising at least 4 double bonds, (for example due to genetic engineering) and which are characterized in that such PUFA(s) is/are produced. However, most preferably, the extract as disclosed and defined herein is derived from non-transgenic organisms, in particular non-transgenic bryophytes or algae. Accordingly, the deriving the extract as disclosed and derived herein from transgenic bryophytes or algae is a less preferred embodiment of the current invention. This meets the demand of some consumers for non-genetically engineered organisms, in particular bryophytes or algae.
However, the extract as employed and provided herein may generally also be derived from single- or multicellular transgenic bryophytes or algae. Particularly, said extract may be derived from single- or multicellular transgenic bryophytes or algae which comprise red algae (rhodoplantae), green algae (chlorophyta), stoneworts (charophyta) and bryophytes. As pointed out above, the source for the extract should be (a) bryophyte/algal cell(s) or an bryophytes or algae (or (a) cell(s) derived from said bryophytes or algae) which comprises a high content of PUFAs or groups derived therefrom comprising at least 4 double bonds and at least 20 C-atoms.

PUFA(s), particularly PUFAs comprising at least 4 double bonds and at least 20 C-atoms may be produced in (a) transgenic bryophytes or algae(s) as employed herein by the use of foreign enzymes encoded by (a) transgene(s), i.e. (a) foreign nucleic acid molecule(s) present in said bryophytes or algae. It is particularly envisaged that the foreign nucleic acid molecules to be introduced enable the host organism to produce the desired PUFA(s) and/or corresponding lipids. A nucleic acid molecule to be introduced into an bryophytes or algae, in order to obtain a transgenic bryophyte or algae as employed herein, may be derived from organisms comprising bacteria, protists, fungi, animals and plants. In a preferred embodiment of the present invention, said nucleic acid molecule(s) may be derived from *Shewanella japonica, Shewanella olleyana, Phaeodactylum tricornufum, Mortierella alpina, Isochlysis galbana, Physcomitrella patens, Thelypteris noveboracensis, Borago offcinalis, Caenorhabditis elegans, Thalassiosira sp., Shewanella sp., Osfreococcus sp.* or *Euglena sp.,* in particular *Euglena gracilis.* Preferably, said transgene/nucleic acid molecule(s) encode(s) for a polypeptide having the activity of a polyketide, an acyltransferase, a cytochrome b5 reductase, a desaturase and/or an elongase. Said desaturase or elongase may, for example, be selected from the group consisting of Δ5-desaturase, Δ6-desaturase, Δ8-desaturase, Δ6-elongase or Δ9-elongase. Enzymes which play a role in the PUFA-biosynthetic pathway and transgenic organisms comprising enzymes for the synthesis of PUFAs are described in WO 00/55330, US 2006/0051847, WO 2008/000277, WO 2004/057001, WO 2005/012316, US 2006/0094092, US 2005/00132442. Synthesis of PUFAs in transgenic oleaginous yeast and enzymes involved in PUFA synthesis, which may be introduced in organisms to modulate PUFA synthesis, are described in US 2005/0136519, US 2005/0266537 and US 200710004016. In US 2005/0100995 the polyketide (PKS) systems for the synthesis of PUFAs from the bacteria *Shewanella japonica* and *Shewanella olleyana* are described. Also described therein are genetically modified plants and microorganisms which may be used for the production of PUFAs. US 7247461 and US 7271315 describes the PUFA polyketide synthase systems from the protist *Schizochytrium.* The transgenic nucleic acid molecule may also encode a cytochrome b5. The transgenes encoding the polypeptides mentioned herein above may be overexpressed and may thus exert their effect on PUFA biosynthesis, in particular increasing the content of PUFAs with more than four double bonds. In an alternative embodiment the synthesis of PUFAs may be modulated by knock-out or knock-down of endogenous genes involved in PUFA-biosynthesis of an organism by RNA silencing, siRNAs, or deletion of a gene or insertions into a gene and the like. Of course, it is envisaged in context of this invention that the increase in PUFA synthesis may be due to the combined use of (a) foreign enzyme(s) and inactivation/deletion of (an) endogenous enzyme(s), i.e. an enzyme encoded by an endogenous, not foreign, nucleic acid molecule.

Methods of generating transgenic organisms, in particular transgenic plants, are well known in the art, see e.g. Hoekema, in: The Binary Plant Vector System, Offsetdrukkerij Kanters B.V., Alblasserdam (1985), Chapter V; Fraley et al., Crit. Rev. Plant Sci. 4 (1993), 1-46, An et al., EMBO J. 4 (1985). 277-287; Chan et al., Plant Mol. Biol. 22 (1993), 491-506; Hiei et al., Plant J. 6 (1994) 271-282; Deng et al., Science in China 33 (1990), 28-34; Wilmink et al., Plant Cell Reports 11 (1992), 76-80; May et al., Bio/Technology 13 (1995), 486-492; Conner and Dormisse, Int. J. Plant Sci. 153 (1992), 550-555; Ritchie et al., Transgenic Res. 2 (1993), 252-265; Wan and Lemaux, Plant Physiol. 104 (1994), 37-48; Vasil et al., Bio/Technology 11 (1993), 1553-1558 ; Ritala et al., Plant Mol. Biol. 24 (1994) 317-325; Spencer et al., Theor. Appl. Genet. 79 (1990), 625-631; Pellegrineschi et al., Genome 45 (2002), 421-430.
In particular, methods for generating transgenic plants synthesizing PUFAs are described in WO 2004/057001, WO 2005/012316 and US 6140486.

The extract as described herein and as employed in context of the present invention is treated with UV-A1 light in accordance with the inventive method. It is particularly intended that this UV light is capable of mildly oxidizing (poly)unsaturated fatty acids or groups derived from (poly)unsaturated fatty acids. In accordance with this invention, PUFAs or groups derived therefrom comprising at least 4 double bonds and at least 20 C-atoms are to be mildly oxidized in order to be capable induce an anti-oxidant stress response.

The terms "(mildly) oxidizing" and "(mildly) oxidized" used herein refer to "mildly oxidizing" or "oxidizing" and "mildly oxidized" or "oxidized", respectively. It is particularly envisaged herein that (poly)unsaturated fatty acids or groups derived from (poly)unsaturated fatty acids comprising at least 4 double bonds are mildly oxidized and are therefore capable to induce an anti-oxidant stress response. The meaning of the terms "oxidized" and in particular "mildly oxidized" as used herein in context of the PUFAs and/or the extract to be activated can readily be deduced by the skilled person based on his common general knowledge and the teaching provided herein. Particularly, these terms refer to oxidized or mildly oxidized lipids, particularly lipids comprising groups derived from (a) PUFA(s) comprising at least 4 double bonds. The mildly oxidized PUFAs or lipids as described and defined herein are, for example, characterized in that they are long-chain oxidation products. In other words, the feature of "mildly oxidizing"/"mildly oxidized", as described herein is, for example, intended to mean that the extracts to be produced by the methods of the invention comprise a sufficient (for example cosmetically/pharmaceutically active) amount of long-chain oxidation products, i.e. mildly oxidized PUFAs and or lipids. In the context of the present invention it is particularly intended that the term "long-chain oxidation products" means that oxygen is inserted into the fatty acid group of the lipid or (poly)unsaturated fatty acid to be activated and/or that the corresponding c-chain of the fatty acid (group) is intact. Preferably, it is intended that the c-chain-shortened or fragmented fatty acid groups of lipids or (poly)unsaturated fatty acids are not more than 50%, 30%, 20% or, preferably 10% [v/v] with respect to the unoxidized starting material comprising the lipids including the fatty acid groups or the (poly)unsaturated fatty acids.

Particularly "mild oxidization" as used in the context of this invention is generally intended to mean every oxidative modification (and subsequent rearrangement to the double bonds) of a (poly)unsaturated fatty acid that leaves the fatty acid chain intact. Preferably, the oxidative modification (and subsequent rearrangement) as described herein leaves the (poly)unsaturated fatty acid chain intact for at least 50%, 60%, 70%, 80% or 90% of the starting material. Accordingly, an "activated extract"/"mildly oxidized extract" in accordance with this invention may particularly comprise at least 50%, 60%, 70%, 80% or 90% (poly)unsaturated fatty acids with intact c-chains or at least 50%, 60%, 70%, 80% or 90% lipids comprising these fatty acids. It is of note that it is most preferred that the (poly)unsaturated fatty acid chain is oxidized, however, not shortened. A person skilled in the art is aware of means and methods for testing the extent of oxidation of a (poly)unsaturated fatty acid, a group derived therefrom or, in particular, a (poly)unsaturated fatty acid which is a fatty acid group of a lipid. Hence, a skilled person is in the position to test whether a (poly) unsaturated fatty acid, group derived therefrom or a (poly)unsaturated fatty acid which is a fatty acid group of a lipid is mildly oxidized. For example, MS or HPLC, particularly HPLC coupled with dual MS (HPLC/MS/MS) may be employed for testing the extent of oxidation.

"Mild oxidation" to be applied in accordance with this invention is particularly delimited from a full/complete or even "over"-oxidation, whereby C-chain breakage (or at least the danger thereof) is much higher and the carbon chains of the PUFAs involved are shortened to an undesired extent. Examples for mildly oxidized PUFAs comprised in the extract of this invention and having an intact C-chain (or lipids comprising such PUFAs) are provided herein (see, for example, 5,6-PEIPC, shown below).

As already mentioned herein, the gist of the present invention is the activation of an extract from organic material in order to provide means for inducing an antioxidant stress response. The extract from organic material to be activated in accordance with this invention comprises at least one PUFA or chemical groups of PUFA having at least 4 double bonds and at least 20 contiguous C atoms. Activation of this extract results in an "activated extract", i.e. mildly oxidized extract capable of inducing an antioxidant stress response. Such an improved, activated extract is, accordingly, obtainable by the method disclosed herein.

The UV-A1 light to be employed in context of the method disclosed herein, i.e. to be used for activating the PUFAs/extract described herein, may also be part of a light having a broader spectrum. In other words, the treatment of the extract as employed in the context of the method disclosed herein may also successfully be performed with light of a broad spectrum, as long as UV-A1 light as described and employed herein is comprised in this light of a broad spectrum. In particular, visible light which comprises UV-A1 light may be employed herein. The term "visible light" is well known in the art. Particularly, visible light has a wavelength from 380 to 750 nm. As pointed out above, the UV light has preferably a high proportion of UVA-1 (or long wave UV light). An example of such a light of a broad spectrum comprising the UV light described and employed herein is sun-light, light employed in a sun bed, and the like. Preferably, the UV light employed herein comprises long wave UV light. Most preferably, said UV light comprises UVA-1. Said UVA-1 has a wavelength from 315 to 400 nm. More preferably, said UVA-1 has a wavelength from 340 to 390 nm. For example, within this range there might be a peak at 350 nm to 352 nm.
It is also envisaged herein that a sensitizer as described herein may also generate singlet oxygen in the presence of light of a different quality, such as long-wave light, e.g. visible light without UV light.

The relevant fluency of the UV light to be applied in context of the method of the present invention can readily be deduced by the skilled person on the basis of the teaching provided herein (for example, on the basis of the present examples). A particular fluency of UV light as applied in context of this invention is a fluency of at least 1 J/cm² to 200 J/cm², in particular at least 5 J/cm² to 100 J/cm², for example of a UV light to be applied herein to the extract to be produced herein. Preferably, said fluency of UV light as applied in context of the present invention may be a fluency of at least 10 J/cm² to 80 J/cm², most preferably, a fluency of 80 J/cm². For example, the fluency applied to the extract can be measured using a IL-1700 radiometer (International Light, Newburyport, MA). The UVA source (Mutzhaas Supersun 5000) used in the present examples was measured at 0.7 mW/cm² at a tube to target distance of 30 cm for the UV (particularly UVA) source.

It is preferred that the extract is treated with UV light which does not comprise medium or short wave UV light, in particular UVB or UVC. In the context of the present invention the term "UVB" means UV light that comprises a wavelength from 280 to 315, whereas the term "UVC" means UV light that comprises a wavelength shorter than 280 nm.
As documented herein, a treatment of the PAPC with UVB does not activate the PAPC, whereas treatment with UVA-1 activates PAPC, see Example 2. In particular, treatment of PAPC with UVA-1 activates PAPC in a dose-dependent manner, whereas treatment with UVB does not activate PAPC, as exemplified in Example 2.

Application of the extract treated with UVA-1 (example of an "activated extract" in context of the present invention) to skin cells leads to an antioxidant response in these skin cells as shown in examples 5 to 8. In view of the above, the extract as employed herein, should, in accordance with this invention, not be treated with light comprising wavelengths longer than 400 nm or shorter than 315 nm. Accordingly, as mentioned, the desired wavelengths of the light to be employed herein are wavelengths from 315 to 400 nm.

One main aspect of the UV light to be employed in the context of the present invention is the feature of its capability of oxidizing, particularly mildly oxidizing lipids as described and defined herein, i.e. lipids comprising at least one group derived from an unsaturated fatty acid or a polyunsaturated fatty acid, preferably a PUFA comprising at least 4 double bonds. The definitions given herein with respect to the terms "oxidizing" and "mildly oxidizing" apply here, mutatis mutandis.

The experimental results summarized in the following show the characterization of 1-palmitoyl-2-(epoxyisoprostane E2)-sn-glycero-3-phosphorylcholine (PEIPC) as an example for a long-chain oxidation product obtainable by mildly oxidizing 1-palmitoyl-2-arachidonoyl-*sn*-glycero-3-phsophorylcholine (PAPC) according to the invention. PAPC is a compound surprisingly .found to be present in an extract from *Physcomitrella patens* as shown in Examples 5 and 6. Treatment of the extract with UVA-1 according to the invention reduces the relative abundance of PAPC.
Example 1 shows that PEIPC is produced by treatment of PAPC with UVA-1; Example 1 also shows that long-chain oxidation products such as PEIPC but not fragmented oxidation products (SC) derived from PAPC and polar phospholipids (PO) such as dimyristoyl-phosphorylcholine (DMPC), 1-palmitoyl-2-(5-oxovaleroyl)-sn-glycero-3-phosphorylcholine (POVPC), 1-palmitoyl-2-glutaroyl-*sn*-glycero-3-phosphorylcholine (PGPC) and 1-palmitoyl-2-lyso-glycero-3-phosphorylcholine (LysoPC) induce an antioxidant response exemplified by the expression of Heme oxygenase 1.
Examples of fragmented, undesired oxidation products derived from PAPC are shown in the following figure:

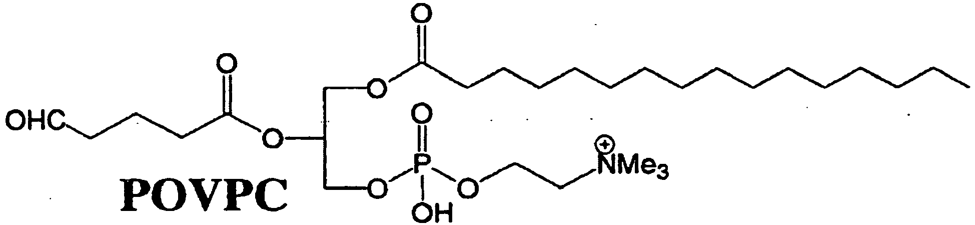

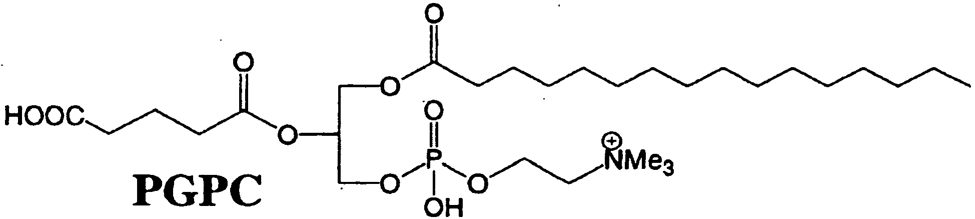

It is also envisaged that the extract disclosed and obtained by the method as disclosed herein, is employed in cosmetic or pharmaceutical compositions. Yet, less preferably but envisaged is the use of a non-mammalian extract of cells or organisms (like moss extract(s)) that are oxidized or may be oxidized by autoxidation, for example in air at room temperature as described in the prior art for chemically synthesized and isolated PUFAs (Ishikawa, J. Clin. Invest. 100, 1209-1216, (1997)). Generally, the extract as described herein may be activated by any kind of oxidizing treatment. Such oxidizing treatments are described in the state of art and can easily be adapted to the method of the present invention by the skilled person taking advantage of the teaching provided herein. It is of note that oxidizing treatments other than the UVA-1 treatment described in this invention might have disadvantages, such as a lower yield of desired (mildly) oxidized PUFAs.

In context of the present invention, the treatment of organic material or an extract as described and employed herein with UV light, autoxidation or a any other oxidation method can be carried out at different points in time and/or at different stages of purification of the organic material/extract, e.g. the points in time/different stages of purification as indicated herein (e.g. in the appended examples). For example, the organic material may be treated with UV light (or by autoxidation or any other oxidation method) directly after the extract was obtained, i.e. the crude extract may be treated. Particularly, a crude extract as described herein may be obtained by mechanical pulping followed by the treatment of said crude extract with UV light. Preferably, a purified extract as employed herein may be treated. Particularly, a purified extract of the organic material is obtained in the presence of isopropanol or any other organic solvent and may then be treated with UV light. More preferably, an extract to be treated with UV light in context of the present invention may be an extract of the organic material obtained in the presence of isopropanol or any other organic solvent followed by drying the extract, i.e. the dried extract is treated with UV light, see also appended examples.
Generally, the person skilled in the art is capable of preparing an extract from (a) non-mammalian cell(s) or a non-mammalian organism by standard techniques. A preferred method of obtaining and treating an extract in accordance with this invention is described in Example 4. This shows that the extract to be treated in accordance with this invention is an extract that is solubilized in a non-organic solvent. Oxidized lipids may be solubilized more easily in an aqueous or non-organic solvent as compared to unoxidized lipids. Therefore, a relative enrichment of oxidized lipids in the solution may be obtained when aqueous or non-organic solvent buffer during extract preparation, by e.g. mechanical pulping, are used. The preparation of the basic extract of non-mammalian cells or organisms may comprise mechanical pulping, sonication, use of mortars and pestles, freeze-thawing cycles, use of blenders (like Waring-Blenders, Polytron), liquid homogenization and maceration (see also appended examples), or e.g. Dounce homogenization, Potter-Elvehjem, French Press etc.

The person skilled in the art is aware of means and methods to prepare cellular extracts or extracts from organism like plants, in particular lower plants like mosses and algae. In the appended examples, a mechanical maceration is used However, the extracts to be treated in accordance with the present invention in order to obtain the mildly oxidized extract(s) of the present invention, in particular the exemplified UVA-1 activated extract from *Physcomitrella patens,* may be obtained by disrupting the cells and cells from the non-mammalian organism to be employed by mechanical/physical or chemical means, like by use of detergents.
The technique chosen for the disruption of cells, whether physical or detergent-based, must take into consideration the origin of the cells or tissues being examined and the inherent ease or difficulty in disrupting their outer layer(s).

Mechanical methods rely on the use of rotating blades to grind and disperse large amounts of complex tissue, such as plant leaves, fronds etc. The Waring blender and the Polytron are commonly used for this purpose. Unlike the Waring blender, which is similar to a standard household blender, the Polytron draws tissue into a long shaft containing rotating blades.

Liquid-based homogenization is the most widely used cell disruption technique for cultured cells. Cells are lyzed by forcing the cell or tissue suspension through a narrow space, thereby shearing the cell membranes. Three different types of homogenizers are in common use. A Dounce homogenizer consists of a round glass pestle that is manually driven into a glass tube. A Potter-Elvehjem homogenizer consists of a manually or mechanically driven Teflon pestle shaped to fit a rounded or conical vessel. The number of strokes and the speed at which the strokes are administered influences the effectiveness of Dounce and Potter-Elvehjem homogenization methods. Both homogenizers can be obtained in a variety of sizes to accommodate a range of volumes. A French press consists of a piston that is used to apply high pressure to a sample volume of 40 to 250 ml, forcing it through a tiny hole in the press. Only two passes are required for efficient lysis due to the high pressures used with this process. French press is often the method of choice for breaking bacterial cells mechanically. It is of note that in more industrial applications also other, larger devices may be employed the prepare the extracts from cells and organisms to be treated in accordance with the present invention, in order to obtain a the mildly oxidized extract(s) of the present invention, in particular the exemplified UVA-1 activated extract from *Physcomitrella patens.*

Sonication is also a physical disruption commonly used to break or open up cells. The method uses pulsed, high frequency sound waves to agitate and lyse cells, bacteria, single-cell algae, mosses, spores and finely diced tissue. To prevent excessive heating, ultrasonic treatment may be applied in multiple short bursts to a sample immersed in an ice bath. Sonication is best suited for volumes <100 ml.

The freeze/thaw method is commonly used to lyse bacterial and cells from higher organism. The technique involves freezing a cell suspension in a dry ice/ethanol bath or freezer and then thawing the material at room temperature or 37°C. This method of lysis causes cells to swell and ultimately break as ice crystals form during the freezing process and then contract during thawing. Multiple cycles are necessary for efficient lysis, and the process can be quite lengthy..

Cells, organisms as well as tissue may treated with various agents to aid the disruption process. Lysis can be promoted by suspending cells in a hypotonic buffer, which cause them to swell and burst more readily under physical shearing. Lysozyme can be used to digest the polysaccharide component of yeast and bacterial cell walls. Alternatively, processing can be expedited by treating cells with glass beads in order to facilitate the crushing of cell walls. Viscosity of a sample typically increases during lysis due to the release of nucleic acid material. DNase may be added to samples along with to reduce this problem.

Less preferred, however envisaged, is the use of detergents in the preparation of the extracts to be treated in accordance with the present invention. Detergents are a class of molecules whose unique properties enable manipulation (disruption or formation) of hydrophobic-hydrophilic interactions among molecules in biological samples. Such detergents may be used to lyse cells, solubilize membrane proteins and lipids. Generally, moderate concentrations of mild (i.e., nonionic) detergents compromise the integrity of cell membranes, thereby facilitating lysis of cells and extraction of soluble protein, often in native form. Using other conditions, detergents effectively penetrate between the membrane bilayers at concentrations sufficient to form mixed micelles with isolated phospholipids. Detergents may be, e.g. Triton X-100^{®}, Triton-X-114^{®}, NP-40^{®}; CHAPS, Tween-20^{®}, Tween-40^{®}, Tween-80^{®}, Octyl Glucoside, Octylthio Glucoside, Brij-35, Brij-58, SDS and the like. Yet, as pointed out herin above, in context of the present invention it is most preferred that any additional chemical treatment of the basic extract be avoided and that mechanically/physically disrupted cells/tissues/organisms are employed as basic extract. However, it may be useful to stabilize the extract by certain chemical means. Illustrative stabilizers are discussed herein below in context of pharmaceutical or cosmetic compositions.

The cells and organism to be employed in order to obtain the basic extract may be cells of natural origin as well as cultured cells or organisms. In context of the present invention, the term "cell" and "organism" to be used as basic material for preparing the extract to be treated by the method of the present invention also comprises the use of "tissues". Such tissues may be leaves, sprouts, roots or reproductive organs e.g. flowers, in particular if these tissues or parts of these tissues contain chlorophyll as well as a given, preferably high proportion of PUFAs, preferably PUFAs or groups derived therefrom comprising at least 4 double bonds. In addition, callus or cell cultures may be used which may be derived from tissues such as leaves, sprouts, roots, reproductive organs, e.g. flowers, or parts thereof and which are grown in liquid culture or on solidified culture medium. The appropriate culturing methods of calli or cell cultures are known to a person skilled in the art. A culture medium may be for example a MS (Murashige and Skoog) medium while a solidifying agent may be agarose, plant agar or bacto agar. A basic culture medium such as a MS medium may be modified in respect to pH range, carbon or nitrogen source, amino acids or vitamins amongst others. The use of plants regenerated from such callus or cell cultures is also envisaged, as well as plants or organisms generally grown or propagated in vitro.

Example 2 shows that an antioxidant response exemplified by mRNA expression of Heme oxygenase 1 can be induced by application of oxidation products of PAPC which had been treated with increasing fluencies of UVA-1. Particularly, it is shown that applying oxidized products obtainable by the treatment of PAPC with UVA-1 (until a fluency of at least 10 J/cm²) is sufficient to induce a minimal antioxidant response whereas the application of oxidized products obtainable by the treatment of PAPC with UVA-1 (until a fluency of maximal 80 J/cm²) induces the maximal antioxidant response in context of this example.

As mentioned above, the extract from organic material described and provided in the context of the present invention may be a crude extract or any other kind of "extract" known in the art. Preferably, said extract from organic material is a purified extract. "Purified extract" in the context of the present invention means that lipids comprised in said extract are enriched, for example, with respect to the crude extract defined herein. "Enriched" in this context may be at least a 5%, 10%, 20%, 30%, 50%, 70%, 100%, threefold, fivefold, tenfold or at least fifty fold higher (e.g. in terms of concentration), for example as compared to the crude extract. In the context of the present invention, "purified extract" can also mean that undesired or disturbing compounds, like debris (for example, compounds of the cells wall, and the like), are excluded/eliminated from the extract. Based on the teaching provided herein, the skilled person is readily in the position to deduce whether a given compound is undesired/disturbing the means and methods of the present invention.

In a preferred, embodiment of the present invention a crude extract may be an aqueous extract. Preferably, as already mentioned above, said crude extract is obtainable by mechanical pulping. The purified extract described in the context of the present invention may be purified for example according to the methods described herein, e.g. in the appended examples. Preferably, said purified extract may be obtainable by purification using organic solvents, such as disclosed in Example 4.
As mentioned, the "extract" in context of this invention comprises a certain, preferably a high amount of (a) (poly)unsaturated fatty acid(s), in particular PUFAs or groups derived therefrom comprising at least 4 double bonds. The "extract" may additionally comprise (a) sensitizer(s), like (a) sensitizer(s) described herein.

The method of the current invention as well as a corresponding activated extract is in detail provided in exemplary fashion in the appended examples. The extract may be obtained by a lipid extraction method as described in Example 4. In this example tissue is washed with phosphate buffered saline supplemented with 0.5 mM EDTA (pH 8.0) and minced and macerated in the presence of isopropanol. Additionally, the samples may be shortly (e.g. from 5 to 20 min) boiled after iso-propanol extraction to avoid undesired cleavage of the lipids caused by lipases present in plants. (Phospho)lipases, especially phospholipase D, are activated by alcohols or diethyl ethers present in extraction mixtures and may be inactivated by boiling. Alternatively, the inactivated plant tissue may be extracted with ethanol-diethyl ether (3:1, v/v) instead of isopropanol followed by vortexing. The tissue may also be extracted with chloroform- isopropanol (7:11, v/v) followed by intensive shaking for 2 hrs.
After filtration the extract is evaporated and subsequently dissolved in chloroform/methanol. After adding PBS, chloroform, chloroform/methanol or formic acid, the organic phase is separated and the organic solvent is removed. The dried material is then solubilized in an organic solvent, preferably chloroform. Aiternatively, any organic solvent can be used instead of isopropanol, wherein organic solvents that do not increase lipid oxidation are preferred. Organic solvents that increase lipid oxidation, for example ethanol, methanol, tetrahydrofuran or diethylether are less preferred. Storage of the dried material before solubilizing the material in an organic solvent may increase lipid oxidation.

The extract can also be obtained by alternative extraction methods known in the art and adaptable for the means and methods of the present invention by one skilled in the art.

Further alternative extraction methods may be: method after Bligh and Dyer (Can. J. Biochem. Physiol. 37, 911-917 (1959)), method according to Nicols (Biochim. Biophys. Acta. 70, 417-422 (1963) and Christie (Lipid analysis 3rd Ed., The Oily Press, Bridgewater, UK (2003)), method according to Hanson and Lester (J. Lipid Res. 21, 309-315 (1980)), method according to Toledo (J. Med. Vet. Mycol. 33, 247-251 (1995)), soxhlet extractions (Soxhlet standard, Soxhlet warm, hot extraction and continous extraction, microwave-assisted extraction), ultrasound-assisted extraction (Ruiz-Jimenez, Anal. Chim. Acta 502, 75-82, (2004)), supercritical fluid extraction (Tanaka , J. Oleo Sci. 53, 417-424 (2004)), pressurized fluid extraction (Toschi Food Chem. 83, 551-555 (2003); Moreaua et al., JAOCS, 80, 1063-1067 (2003)), microwave oven extraction (Ganzler J. Chromatogr. 371, 299-306 (1986); Leray , Analusis 23, 65 (1995)).

Preferably, the mildly oxidized extract of the present invention, in particular the exemplified UVA-1 activated extract from *Physcomitrella patens,* produced in accordance with the inventive methods may be a lipid extract. However, it is explicitly not excluded that the extract comprises other compounds besides lipids that can be activated by the treatment with UV light, autoxidation, in particular in the presence of metals such as copper or iron, enzymatic peroxidation using enzymes like lipoxygenase, cyclooxygenase or fatty acid epoxygenase or any other kind of oxidizing treatment as described herein. In particular, it may be possible that other activated compounds enhance the effect of activated lipids, for example in a more than additive way, or exhibit the same, or even better effect than the activated lipids. As mentioned, the extract may also comprise sensitizers.

The lipid extract to be employed herein may particularly comprise (a) lipids(s) that comprise(s) at least one group derived from a PUFA comprising at least 4 double bonds. Even more preferably said lipid extract from organic material comprises (a) phospholipids(s). The carboxylic group of said poly(unsaturated) fatty acid may form an ester bond within the lipid or to the glycerolic backbone of the phospholipid. The terms "lipid" and "phospholipid" are well known in the art. In particular "lipid" refers to a molecule having a glycerolic backbone with at least one and up to three fatty acid groups. Preferably, at least one fatty acid group is a polyunsaturated fatty acid group. The term "phospholipid" particularly refers to a lipid having at least one phosphate group. The term "(poly)unsaturated fatty acid" used herein refers to polyunsaturated fatty acid or unsaturated fatty acids. A polyunsaturated fatty acids employed herein comprises at least 2 double bonds, preferably at least 4 double bonds. An unsaturated fatty acid as described herein comprises at least 1 double bond.

Accordingly, also the "group derived from a polyunsaturated fatty acid" as used herein preferably comprises at least 1 double bond, in particular at least 4 double bonds. The term "group derived from a (poly)unsaturated fatty acid" used herein refers to a chemical group of a (poly)unsaturated fatty acid, in particular a fatty acid group of a lipid. Said group may also comprise at least 20 C atoms. In a preferred embodiment of the present invention, the group derived from a polyunsaturated fatty as employed herein is derived from arachidonic acid. A group derived from a polyunsaturated fatty acid to be employed herein may also be derived from, for example, docosahexeanoic acid (DHA), eicosapentaenoic acid (EPA) and docosapentaenoic acid (DPA). A most preferred phospholipid to be employed herein and/or that is envisaged to be comprised in the extract to be treated in accordance with the present invention is 1-palmitoyl-2-arachidonoyl-sn-glycero-3-phsophorylcholine (PAPC).

In one embodiment of the present invention, the extract from organic material comprises at least one polyunsaturated fatty acid with at least 4 double bonds. The extract from organic material may also comprise at least one (poly)unsaturated fatty acid which comprises at least 20 C atoms. Preferably, said (poly)unsaturated fatty acid is a fatty acid group of a lipid.

Preferably, a "crude extract" as disclosed and described in the context of the present invention comprises at least 0.5 (v/v)% and at the utmost 100 (v/v)% lipid(s) comprising at least one group derived from a (poly)unsaturated fatty acid as defined herein. More preferably, said "crude extract" comprises at least 5 (v/v)% and at the utmost 100 (v/v)% lipid(s) comprising at least one group derived from a (poly)unsaturated fatty acid as defined herein.

Preferably, a "lipid extract" as disclosed and described in the context of the present invention comprises at least 0.5 (v/v)% and at the utmost 100 (v/v)% lipid(s) comprising at least one group derived from a (poly)unsaturated fatty acid. More preferably, the lipid extract comprises at least 5 (v/v)% and at the utmost 100 (v/v)% lipid(s) comprising at least one group derived from a (poly)unsaturated fatty acid.

In a specific embodiment, a "crude extract" as disclosed and described in the context of the present invention comprises at least 0.1 (v/v)% and at the utmost 50 (v/v)% PAPC. Preferably, said "crude extract" comprises at least 0.5 (v/v)% and at the utmost 10 (v/v)% PAPC.

In another specific embodiment, a "lipid extract" as disclosed and described in context of the present invention comprises at least 0.1 (v/v)% and at the utmost 50 (v/v)% PAPC. More preferably, said "lipid extract" comprises at least 0.5 (v/v)% and at the utmost 10 (v/v)% PAPC.

In an alternative embodiment of the present invention, an extract described or to be employed herein from organic material may comprise (a) free unsaturated fatty acid(s) or (a) free polyunsaturated fatty acid(s), preferably a free PUFA comprising at least 4 double bonds. It is of note that the definitions given herein with respect to "a group derived from a (poly)unsaturated fatty acid" apply to the free (poly)unsaturated fatty acid as defined and employed herein, mutatis mutandis and vice versa. Exemplary free (poly)unsaturated fatty acids comprised in an extract may be arachidonic acid, DHA, DPA and/or EPA.

Preferred polyunsaturated fatty acids (comprised in the inventive extract) are arachidonic acid (AA), docosahexaenoic acid (DHA), eicosapentaenoic acid (EPA), adrenic acid (all-cis-7, 10, 13, 16-docosatetraenoic acid), Osbond acid (all-cis-4, 7, 10, 13, 16-docospentaenoic acid) and clupanodonic acid (all-cis-7, 10, 13, 16, 19-docosapentaenoic acid), wherein arachidonic acid is most preferred.

Preferably, an extract described and to be employed herein comprises at least 0.5 (v/v) % and at the utmost 100 (v/v)% unsaturated fatty acids, preferably PUFAs or groups derived therefrom comprising at least 4 double bonds. More preferably, said extract comprises at least 5 (v/v)% and at the utmost 100 (v/v)% (poly)unsaturated fatty acids, preferably PUFAs or groups derived therefrom comprising at least 4 double bonds.

A (poly)unsaturated fatty acid or group derived therefrom as described or to be employed herein may comprise at least 12 C atoms and at the utmost 40 C atoms, more preferably, at least 14 C atoms and at the utmost 30 C atoms, even more preferably, at least 16 C atoms and at the utmost 24 C atoms, and most preferably particularly or at least 20 C atoms.
A polyunsaturated fatty acid or group derived therefrom as described and employed herein may comprise at least 4, 5, 6, 7, 8, 9 or 10 double bonds. Preferably, said polyunsaturated fatty acid or group derived therefrom may comprise at least 4, 5 or 6 double bonds and up to 10 double bonds. More preferably, said polyunsaturated fatty acid or group derived therefrom comprises at least 4 double bonds. A preferred double bond that is comprised in the (poly)unsaturated fatty acids or groups derived therefrom as described or to be employed herein is envisaged to be a *cis* double bond. Thereby it is preferably envisaged that two *cis* double bonds are separated from each other by a single methylene group. Most preferably, a polyunsaturated fatty acid or group derived therefrom comprises 4 double bonds which are separated from each other by a single methylene group.

Particularly, a polyunsaturated fatty acid or group derived therefrom may comprise (a) group(s) derived from an omega-3 fatty acid or omega-6 fatty acid. In the context of the current invention the term "omega-3 fatty acids", for example, means that the last double bond of the polyunsaturated carbon chain of the group derived from the fatty acid is - seen from the carboxyl end - the third last C-C bond. Accordingly, the term "omega-6 fatty acids" means in the context of the current invention that the last double bond of the polyunsaturated carbon chain of the group derived from the fatty acid is - seen from the carboxyl end - the six last C-C bond. Most preferably said group derived from omega-6 fatty acids is derived from all-cis-5,8,11,14-eicosatetraenoic acid (arachidonic acid) and the pospholipid comprising a group derived from arachidonic acid is 1-palmitoyl-2-arachidonoyl-sn-glycero-3-phsophorylcholine (PAPC). A chemical formula of PAPC is given in the figure below: 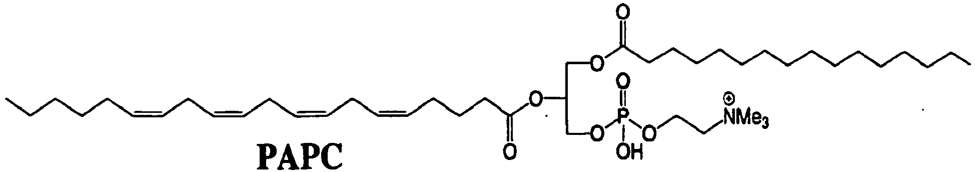 As mentioned above, the extract provided herein or produced in accordance with the inventive methods (and capable of inducing an antioxidant stress response) is, inter alia, a lipid extract. Said lipid extract preferably comprises at least one group derived from a mildly oxidized (poly)unsaturated fatty acid. Said lipid extract is, inter alia, envisaged to comprise (a) (mildly) oxidized lipid(s). Said (mildly) oxidized lipid(s) may comprise at least one group derived from a (mildly) oxidized (poly)unsaturated fatty acid. Preferably, said group derived from a (mildly) oxidized (poly)unsaturated fatty acid may comprise at least 20 C atoms. Said group derived from a (mildly) oxidized (poly)unsaturated fatty acid also may comprise at least 3 O atoms. Particularly, said group derived from a (mildly) oxidized (poly)unsaturated fatty acid may comprise an epoxyisoprostanoid group. Preferably, said epoxyisoprostanoid group is an epoxyisoprostanoid E2 group. Most preferably, said group derived from a (mildly) oxidized (poly)unsaturated fatty acid is derived from all-cis-5,8,11,14-eicosatetraenoic acid (arachidonic acid).
It is preferred in context of the present invention that the extract as described herein comprises (a) phospholipid(s). Preferably, the (mildly) oxidized lipid comprised by said extract is 1-palmitoyl-2-(epoxyisoprostane E2)-sn-glycero-3-phosphorylcholine (PEIPC) as shown in the following figure:

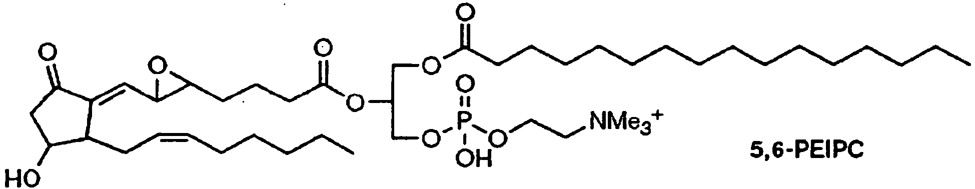

In yet another preferred embodiment of the invention, said phospholipids comprises epoxyisoprostane-containing phosphatidylcholines, e.g. PEIPC as shown above.

In a further embodiment, the present invention relates to (a) mildly oxidized extract(s) of the present invention, in particular the exemplified UVA-1 activated extract from *Physcomitrella patens,* which is capable of inducing an antioxidant stress response as defined herein and to an extract obtainable by the inventive method. The invention also relates to the extract of the present invention for the preparation of a cosmetic composition Also disclosed herein is the extract of the present invention for the preparation of a pharmaceutical composition. The extract capable of inducing an antioxidant stress response as defined herein or obtainable by the inventive method for the treatment, amelioration or prevention of a disorder or a disease which is related to oxidative stress is also envisaged. One embodiment of the present invention relates to said extract for the treatment, amelioration or prevention of symptoms of ageing which are related to oxidative stress.

The invention further relates to the cosmetic use of the extract capable of inducing an antioxidant stress response for the preparation of a cosmetic composition for the cosmetic treatment, amelioration or prevention of symptoms of ageing which are inducible by or related to oxidative stress. The symptom of ageing may be selected from the group consisting of skin ageing, wrinkle formation and saggy skin.

The term "symptoms of ageing which are inducible by or related to oxidative stress" as used herein not only refers to symptoms occurring in elderly people but also in younger people, including adults, young adults or adolescences.
This means that "symptoms of ageing which are inducible by or related to oxidative stress" are not limited to a certain age. As examples, these symptoms are such which are due to the use/abuse of alcohol, caffeine, theine or other drugs, symptoms that are due to dehydration of the skin, symptoms that are due to short of sleep and/or other symptoms that are inducible by or related to oxidative stress.

The use of said extract of the present invention for the preparation of a pharmaceutical composition for the treatment, amelioration or prevention of a disorder or a disease which is inducible by or related to oxidative stress is also envisaged. The invention further describes the use of said extract(s) of the present invention for the treatment, amelioration or prevention of a disorder or a disease which is related to oxidative stress. Furthermore, the invention relates to a cosmetic or pharmaceutical composition comprising the extract of the present invention.

The invention also relates to the non-medical use of the activated extract of the present invention; i.e. the extract obtainable by the inventive method for the cosmetic treatment, amelioration or prevention of symptoms of ageing which are inducible by or related to oxidative stress. Also described herein is a method of treating, ameliorating or preventing a disorder or disease inducible by or related to oxidative I stress comprising administering to a subject in need of such a treatment, prevention or amelioration the activated extract; i.e. the extract obtainable by the herein provided method.

The medical and pharmaceutical use of the mildly oxidized extract, in particular the extract treated with UVA-1 and being derived from the moss *Physcomitrella patens,* provided and described herein for the treatment, amelioration or prevention of a disorder or disease which is inducible by or related to oxidative stress is disclosed herein. Such disorders/diseases to be treated, ameliorated or prevented in accordance with the present invention, may particularly be selected from the group consisting of: sunburn, skin ageing, clinical or severe sunburn, cancer, in particular skin cancer, a disease related to transplantation medicine, inflammatory diseases, psoriasis, atopic dermatitis, cutaneous T-cell lymphoma, eczema such as dyshidrotic hand eczema, cutaneous mastocytosis, connective tissue diseases, lupus erythematodes (localized, systemic), localized scleroderma, localized scleroderma, polymorphic light eruption, mycosis fungoides, urticaria pigmentosa, sclerodermatous graft-versus-host disease, extragenital lichen sclerosus, acrosclerosis in systemic sclerosis, vesicular hand dermatitis, keloid scars, pityriasis lichenoides, granuloma annulare, sarcoidoisis, idiopathic follicular mucinosis, hypereosinophilic syndrome, pityriasis rubra pilaris, alopecia areata, lichen planus, acne vulgaris and vitiligo. However, also the treatment of other disorders, whether benign or malignant is envisaged and part of this invention, like the treatment of verruca vulgaris, verruca plana, condyloma and epidermodysplasia verruciformis as well as the treatment of skin cancer, as will be detailed herein below. Also further, non-limiting disorders will be discussed herein below and are to be treated with the mildly oxidized extract(s) of the present invention, in particular the exemplified UVA-1 activated extract from *Physcomitrella patens.*

As pointed out above, the particular envisaged use of the mildly oxidized extract as described herein, in particular the UVA-1 activated extract from *Physcomitrella patens,* is the cosmetic use or the use as a "cosmeceuticle". The examples appended here document the surprisingly positive anti-oxidant effect of the mildly oxidized extract of the present invention, as compared to non-oxidized (non-UVA-1 treated extract) and as compared to isolated, chemically synthesized PUFAs (non-oxidized PAPC) and oxPAPC/PEIPC. Also it could surprisingly be shown that commercially available plant oils (like corn oil, olive oil, pumpkin seed oil etc.) have no effect on skin cells, even when "activated" by UVA-1 light.

Since the mildly oxidized extract(s) as described herein and/or obtained by the method of the present invention particularly effective in their anti-oxidative advantages, their use in cosmetic products or as cosmetic products is envisaged and part of this invention. Therefore, the mildly oxidized extract (in particular, an activated *Physcomitrella patens* extract) produced in accordance with the inventive methods may be part of or may be a component of skin care products/skin cream compositions such as day- or night creams, anti-ageing creams, anti-ageing lotions, anti-ageing balms, anti-dandruff agents/lotions/shampoos/gels, hair and/or scalp products like shampoos, or sun protection products, like sun creams, sun screen agents, sun lotions, sun (lip) balm, sun gels, après lotions, après creams, après milks etc. The mildly oxidized extract may also be part of or may be a component of toiletry products, such as creams, gels, balms, lotions, ointments, lip balms, (nail) polishers, make-up, mascara products, concealers (for, e.g., covering pimples, spots and the like), hair colours, hair-sprays, hair-gels, deodorants, bath oils, or physical and chemical peels (for, e.g. microdermabrasion).

Said cream composition, oil compositions, lotions, gels, balms and the like provide for a significant anti-ageing effect for, e.g. and in particular the skin and may thus ameliorate negative or undesired effects caused by or leading to modifications of cells and tissues, like, e.g. non-or preclinical sunburn, skin ageing, wrinkle formation/wrinkling, saggy skin, dryness, or age spots. Accordingly, undesired modifications of the skin comprise also symptoms of ageing. Generally, skin changes associated with ageing can also be cosmetically treated in accordance with the present invention. The anti-ageing effect may comprise improved smoothness, reduced wrinkling and reduced spottiness and an increase in firmness and moisture content of the skin.

The basis for such skin care products, in particular creams are known in the art and such a composition may comprise: water, and emulsified and dispersed in the water the inventive extract described herein and/or produced in accordance with the methods as disclosed in this invention. Said extract may comprise at least 10 to 100 µpg/ml (0.001% to 0.01% w/v) of the skin cream composition. More preferably, said extract comprises at least 15 µg/ml to 40 µg/ml (0.0015% to 0.004% w/v) of the skin cream composition. Most preferably, said extract comprises at least 20 µg/ml (0.002% w/v) of the skin cream composition. The solubilization of lipids is well known in the art, see DE utility patent Nr. 20 2005 007 603.1 and may correspondingly applied to the inventive extract.

The mildly oxidized, in particular UV-activated extracts as described herein and as obtained by the method of the present invention may find its use in pharmaceuticals but also and foremost in cosmetics and "cosmeceuticals". As known in the art, most cosmetics (as well as pharmaceuticals or cosmeceuticles) contain a combination of at least some of the following ingredients: water, surfactants, in particular emulsifiers, cosurfactants, preservatives (such as para-hydroxybenzoic acid esters), thickener, colour or colourants, fragrance and stabilizers, like ph stabilizers/regulators, base, gelling agents, oils, waxes, silicone elastomers, organic filters, butylene glycol, osmotic pressure modifiers, fillers, colloidal dispersion of inorganic fillers, soft-focus powders (like, e.g. polyamides, silica, talc, mica, fibres, in particular polyamide or cellulose), tightening agents, plant proteins, synthetic latexes (in particular acrylic), sequestering agents (e.g. salts of EDTA), UV-A and UV-B screening agents, antioxidants (like e.g. [alpha]-tocopherol, butylhydroxy- anisole or butylhydroxytoluene, superoxide dismutase, ubiquinol or certain metal chelators, depigmenting agents, such as hydroguinone, azelaic acid, cafeic acid or kojic acid emollients; moisturizers (like, e.g. polyols including glycerol, PEG 400, or thia-morpholinone, and its derivatives, mucopolysaccharides such as hyaluronic acid at its salts and esters, or urea), wetting agents, moisture regulators, antiseborrhoeic or anti-acne agents, (like, e.g. S-carboxymethylcysteine, S-benzylcysteamine, their salts or their derivatives, or benzoyl peroxide).

A person skilled in the art may deduce further ingredients contained in a cosmetic composition for example from the CTFA Dictionary (International Cosmetic Ingredient Dictionary and Handbook published by the Cosmetic, Toiletry and Fragrance Association, 9th Edition, 2002).

In a pharmaceutical use of the mildly oxidized extract(s) of the present invention, in particular the exemplified UVA-1 activated extract from *Physcomitrella patens,* additional medically active components or compounds may be employed. Such compounds may be, e.g. antibiotics. Antibiotics may also be an additional ingredient in the cosmetic or cosmeceutical compositions of the present invention. These antibiotics may comprise or may be erythromycin and its esters, neomycin, clindamycin and its esters, tetracyclines, etc. These are examples. Also antifungal agents, such as ketoconazole or 4,5-polymethylene-3∼isothiazolidones, may be part of the inventive pharmaceutical or cosmeceutical composition.

examples of oils are synthetic oils like fluorinated oils, alkyl benzoates and branched hydrocarbons such as polybutene, volatile or non-volatile oils, linear or cyclic silicone oils (e.g. dimethylpolysiloxanes (dimethicones), polyalkylcylcosiloxanes (cyclomethicones) or polyalkylphenylsiloxanes (phenyldimethicones)), vegetable oils, such as soybean oil, jojoba oil and the like, and mineral oils such as paraffin oil. Exemplary waxes may be beeswax, polyethylene wax, carnauba wax or ozocerite.

Other components of the inventive pharmaceutical or cosmetic or cosmeceutical composition are, inter alia, agents promoting hair regrowth, such as Minoxidil (2,4-diamino-6-piperidinopyrimidine 3-oxide) and its derivatives, Diazoxide (7∼chloro-3-methyl-I, 2,4-benzothiadiazine 1,1-dioxide) and Phenytoin {5,4-diphenylimidazolidine-2,4-dione); nonsteroidal anti-inflammatory agents; carotenoids, for example [beta]-carotene; or antipsoriatic agents such as anthralin and its derivatives

Commonly used emulsifiers comprise : mono- and distearate esters of polyoxyethylene and free polyethylene oxide, partial esters of lauric, palmitic, stearic and oleic acids and hexitol anhydrides and 120-mole ethoxylated jojobaoil.

Also silicone elastomers may be ingredients of a cosmetic composition. Such elastomers can be obtained, for example, by reacting a polysiloxane having at least one reactive group (in particular hydrogen or vinyl) and carrying at least one end and/or side alkyl (particularly methyl) or phenyl group with an organosilicon such as an organohydrogenpolysiloxane in the presence of a catalyst.

Organic filters to be used in cosmetic compositions comprise, derivatives of dibenzoylmethane, derivatives of cinnamic acid, salicylates, para-aminobenzoic acids, benzophenones, derivatives of benzylidene comphor, phenylbenzimidazoles, triazines; phenylbenzotriazoles and anthranilic derivatives.

Exemplary surfactants are esters of fatty acids and polyols, for example esters of fatty alcohols and PEG, esters of fatty acids and sucrose, esters of fatty acids and sorbitan, esters of fatty acids and glycerol. Surfactants to be used in a cosmetic composition may also be alkylpolyglucosides, modified polysiloxane polyethers, betaine and its derivatives, polyquaterniums, sulphate salts of ethoxylated fatty alcohols, sulfosuccinates, sarcosinates, alkyl- and dialkylphosphates and their salts and soaps of fatty acids. Non limiting examples of cosurfactants are linear fatty alcohols, particularly stearyl and hexadecyl alcohols.

Preservatives used in cosmetics as well as pharmaceuticals and cosmetic or cosmeceuticals may include parabens, benzyl alcohol and tetrasodium EDTA (ethylenediaminetetra-acetic acid) and the like. Thickening agents such as polymers are often added to cosmetics to change their consistency. Exemplary thickeners are xanthan or guar gum, cellulose derivatives, silicone gums (dimethiconol) and hydrophilic or amphiphilic, crosslinked or non-crosslinked homo- and copolymers of acrylamidoethylpropane sulfonic acid (AMPS) and/or of acrylamide and/or of acrylic acid and/or of salts or esters of acrylic acid. Polymers can be synthetic (e.g, polyethylene glycol) or derived from natural sources (like polysaccharides). Seaweeds are a common source of natural polysaccharides - carrageenans are extracted from red algae and alginates from brown algae. Cosmetics that are too thick can be diluted with solvents such as water or alcohol.

Exemplary cosmetic compositions such as gels comprise gelling agent 0.50%, base 0.38%, preservative 0.40%, the inventive extract 0.50% and water (without minerals) 98.12%. Similarly, other exemplary cosmetic compositions such as oil in water emulsions, water in oil or multiple emulsions (e.g. water/oil/water or oil/water/oil) comprise butylene glycol 5.0%, gelling agent 0.2%, emulsifier 4.0%, co-emulsifier 0.2%, preservative 1.0%, oils 8.0%, antioxidant 0.3%, base 0.2%, ph adjuster 0.1%, the inventive extract 0.5% and water (without minerals) 80.5%. The content of each ingredient is expressed in percent by weight/weight. The methods to produce such cosmetic compositions are well known to a person skilled in the art. The emulsions may also be microemulsions or nanoemulsions. Preferably, the composition is an oil in water emulsion.

Accordingly, the present invention does not only provide for a method or a process of preparing a highly active, mildly oxidized extract of non-mammalian cells or organisms (in particular of a UVA-1 activated extract from mosses, like *Physcomitrella patens)* but also provides for composition wherein said activated/mildly oxidized extract(s) is/are used, for example in pharmaceutical compositions for use in human or veterinary medicine, or else in cosmetic compositions.

The mildly-oxidized extracts according to the invention are particularly suitable in the following treatment fields: for treating dermatological, in particular for treating common acne, comedone-type acne, polymorphic acne, acne rosacea, nodulocystic acne, acne conglobata, senile acne, secondary acne such as solar acne, acne medicamentosa or occupational acne; for treating other types of keratinization disorders, in particular ichthyosis, ichthyosiform states, and the like; for treating other dermatological conditions with an inflammatory immunoallergic component, with or without cell proliferation disorder, and in particular all the forms of psoriasis, whether cutaneous, mucosal or ungualor cutaneous atopy, such as eczema or respiratory atopy or gingival hypertrophy; for treating any dermal or epidermal proliferations whether benign or malignant, whether of viral or non- viral origin, such as verruca vulgaris, verruca plana, condyloma and epidermodysplasia verruciformis, for treating other dermatological disorders such as immune dermatoses such as lupus erythematosus, bullous immune diseases and collagen diseases, such as scleroderma. A particular envisaged use is the pharmaceutical use in the prevention and treatment of skin disorders due to exposure to UV radiation and for repairing or combating skin ageing, whether photoinduced or chronological, or any pathologies associated with chronological or actinic ageing, such as xerosis. As mentioned above, also the pharmaceutical (as well as cosmetically use in the management of sunburns (normal, severe and clinical) is envisaged and part of this invention. Yet, also the treatment of disorders like skin cancer, acute inflammatory diseases, graft-versus-host disease etc. is envisaged.

The pharmaceutical or cosmetic composition of the invention comprises, in a physiologically acceptable medium or per se, the mildly-oxidized extract(s) according to the invention. The compositions according to the invention therefore comprise at least 0.001% of the mildly-oxidized extracts according to the invention in combination with a physiologically acceptable support or at least one acceptable excipient, chosen according to the desired cosmetic or pharmaceutical form and the chosen method of administration. The expression "physiologically acceptable medium or support" is intended to mean a medium or a support that is compatible with the skin, the mucous membranes and/or the integuments. The term "pharmaceutically acceptable excipient" is intended to mean a substance which is inert with respect to the components comprised in the mildly-oxidized extracts according to the invention, and compatible with the skin, but also with mucous membranes and/or the integuments. The administration of the composition according to the invention, either pharmaceutical or cosmetic, may be carried out topically. Yet, it is also envisaged that the mildly-oxidized extracts according to the invention may be used enterally or parenterally. Preferably, the pharmaceutical composition is packaged in a form suitable for topical application.

When administered topically, the pharmaceutical or cosmetic composition according to the invention is more particularly for use in the treatment of the skin or in skin-care and treatment or care of the mucous membranes. These pharmaceutical or cosmetic composition may be in the form of salves, creams, milks, ointments, powders, impregnated pads, syndets, solutions, gels, sprays, mousses, suspensions, lotions, sticks, shampoos or washing bases. Also envisaged are suspensions of lipid or polymeric microspheres or nanospheres or vesicles or of polymeric patches and of hydrogels.

In one embodiment the mildly oxidized extract(s) of the present invention, in particular the exemplified UVA-1 activated extract from *Physcomitrella patens,* is/are used topically at a concentration generally of between 0.001% and 50% by weight, preferably between 0.01% and 20% by weight, relative to the total weight of the composition. The mildly oxidized extract(s) of the present invention, in particular the exemplified UVA-1 activated extract from *Physcomitrella patens,* according to the invention may also be useful in the following cosmetic areas (besides being particular useful in anti-aging creams/ointments, etc) body hygiene, hair care, make-up, peeling solutions, systems to prevent first signs of age, anti-wrinkle formulations or formulations increasing skin firmness.

Cosmetic composition according to the invention may contain, e.g. in a cosmetically acceptable support, the mildly oxidized extract(s) of the present invention, in particular the exemplified UVA-1 activated extract from *Physcomitrella patens* or *Thelypteris noveboracensis* or other extracts as disclosed herein (like, for example, fish oil). These cosmetic compositions may be, as pointed out above, a milk, a cream, a lotion, a gel, suspensions of lipid or polymeric microspheres or nanospheres or vesicles, impregnated pads, solutions, sprays, mousses, sticks, soaps, shampoos or washing bases and the like.

In the cosmetic use, the cosmetic composition comprises, in a physiologically acceptable support, the mildly oxidized extract(s) of the present invention, in particular the exemplified UVA-1 activated extract from *Physcomitrella patens* or from fern(s) or from fish oil, in its pure (activated) form, as a crude lipid extract, as a purified extract, as a solubilized extract and/or as a reconstituted extract (for a example in an aqueous extract) as defined above. However, it is also envisaged that the mildly oxidized extract(s) of the present invention, in particular the exemplified UVA-1 activated extract from *Physcomitrella patens* or from fern(s) or from fish oil, is (after its activation by UV-light) dried before it is used in the cosmetic or pharmaceutical composition provided herein.
The present invention is further described by reference to the following figures and examples.

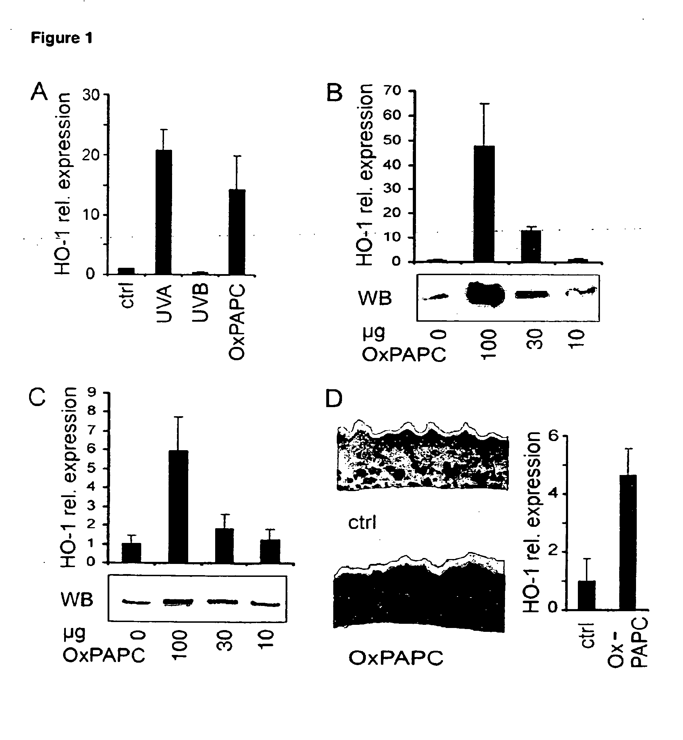

The Figures show
- **Figure 1:**: **OxPAPC induces HO-1 expression in skin cells**
*A*, levels of HO-1 mRNA expression in dermal fibroblasts 4h after stimulation with 40 J/cm² UVA-1, 20 mJ/cm² UVB or 100 µg/ml OxPAPC. Relative expression of HO-1 mRNA [fold induction over control] was determined using quantitative real-time PCR (qPCR) normalized to expression of the housekeeping gene beta-2-microglobulin (B2M). Data represent means +/- S.D. *B,C,* Keratinocytes *(B)* and dermal fibroblasts *(C)* were incubated with the indicated concentrations (µg/ml) of OxPAPC and the induction of HO-1 mRNA was measured after 4 h by qPCR. The inserts show corresponding HO-1 protein levels as determined by Western blot after 6 h of treatment. (*D*) Primary keratinocytes differentiated in a three-dimensional epidermal equivalent were incubated with OxPAPC at the indicated concentrations for 4 days and analyzed for HO-1 expression using immunohistochemistry and qPCR.

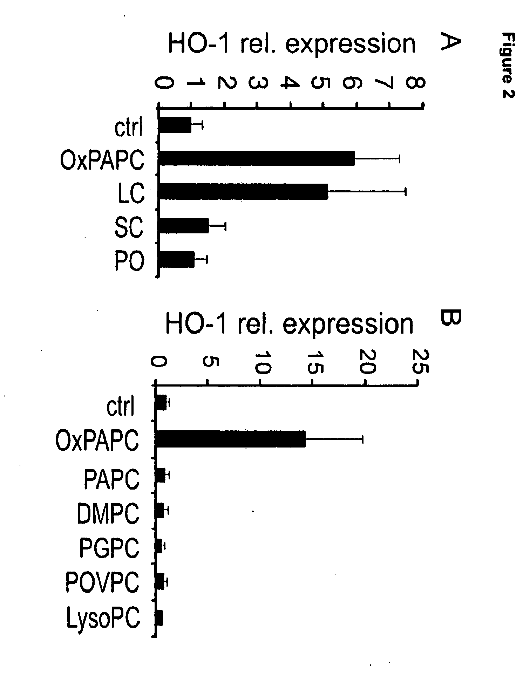

- **Figure 2:**: **Long chain, but not fragmented, oxidized phospholipids contain HO-1-inducing activity**
(A) Autoxidized PAPC (oxPAPC) as well as long chain oxidation products (LC) separated from oxPAPC but not fragmented oxidation products (SC) and polar phospholipids (PO) separated from oxPAPC induce Heme Oxygenase 1 (HO-1) mRNA expression in dermal fibroblasts relative to non-treated control cells (ctrl).
(B) Autoxidized PAPC (oxPAPC) but not PAPC, dimyristoyl-phosphorylcholine (DMPC), 1-palmitoyl-2-(5-oxovaleroyl)-*sn*-glycero-3-phosphorylcholine (POVPC), 1-palmitoyl-2-glutaroyl-*sn*-glycero-3-phosphorylcholine (PGPC) and 1-palmitoyl-2-lyso-glycero-3-phosphorylcholine (LysoPC) induce Heme Oxygenase 1 (HO-1) mRNA expression in dermal fibroblasts relative non-treated control cells (ctrl). Relative expression of HO-1 mRNA [fold Induction over control] was determined using quantitative real-time PCR (qPCR) normalized to expression of the housekeeping gene beta-2-microglobulin (B2M). Data represent means +/- S.D.

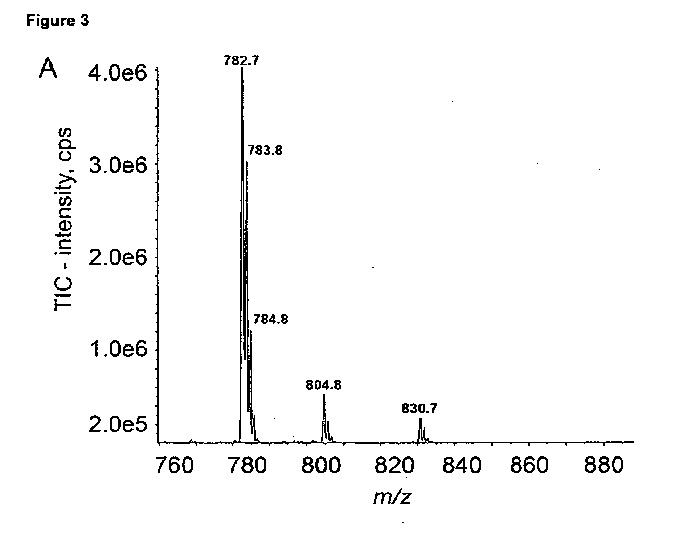

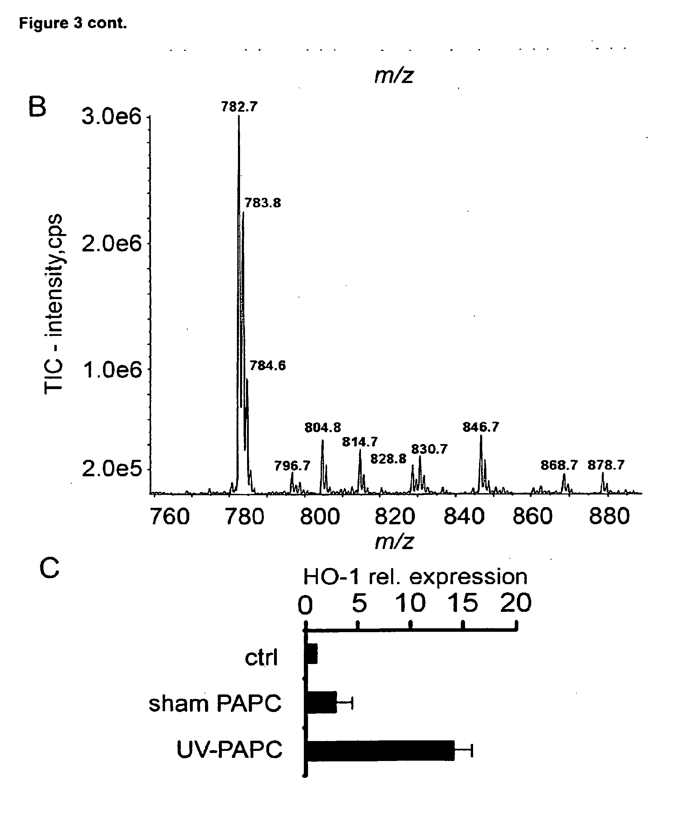

- **Figure 3:**: ***In vitro* irradiation of PAPC with UVA-1 leads to formation of Heme Oxygenase inducing oxidation products**
(A) ESI-MS (positive mode, *m*/*z* range from 760 to 900 Da shown) of sham treated PAPC.
(B) ESI-MS (positive mode, *m*/*z* range from 760 to 900 Da shown) of UVA-1 treated PAPC.
(C) shows Heme Oxygenase (HO-1) mRNA expression 4h after treatment with sham treated and UVA-1 treated PAPC relative to non-treated control cells (ctrl).
Relative expression of HO-1 mRNA [fold induction over control] was determined using quantitative real-time PCR (qPCR) normalized to expression of the housekeeping gene beta-2-microglobulin (B2M). Data represent means +/- S.D.

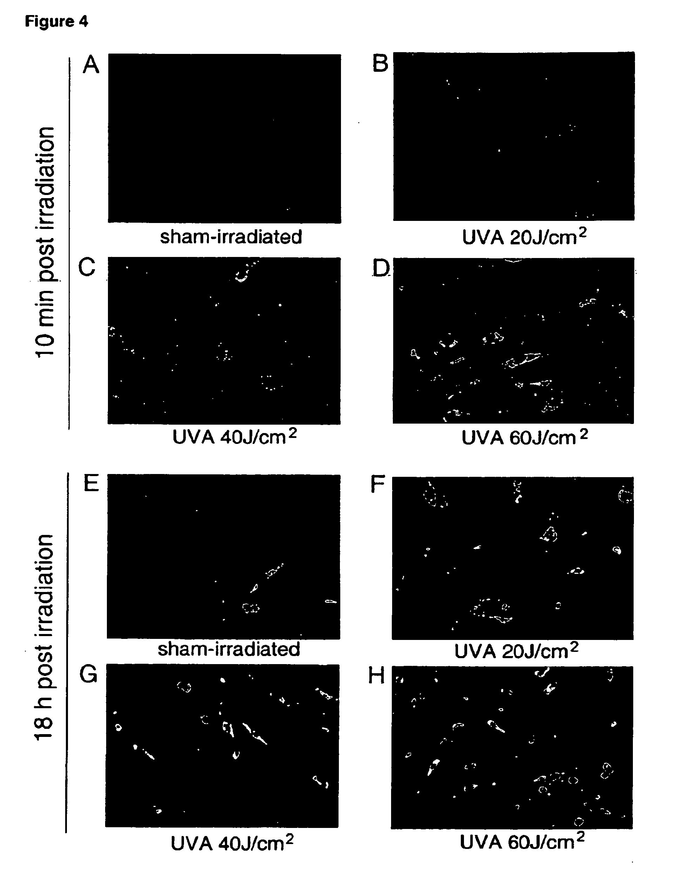

- **Figure 4:**: **UVA-1 irradiation of dermal fibroblasts leads to intracellular formation of oxidation specific epitopes of PAPC**
(*A,B,C,D*), dermal fibroblasts cultured in 8 well chamber slides were subjected to increasing fluencies of UVA-1 (sham (*A*), 20 J/cm² (*B*), 40 J/cm² (*C*) and 60 J/cm² (*D*)), immediately fixed and stained with EO6, IgM monoclonal mouse antibody. (*E,F,G,H*) Dermal fibroblasts cultured in 8 well chamber slides were subjected to increasing fluencies of UVA-1 (sham (*E*), 20 J/cm²(*F*), 40 J/cm² (*G*) and 60 J/cm² (*H*)), cultured for 18h, fixed and stained with EO6 antibody and an antibody detecting active Caspase 3.

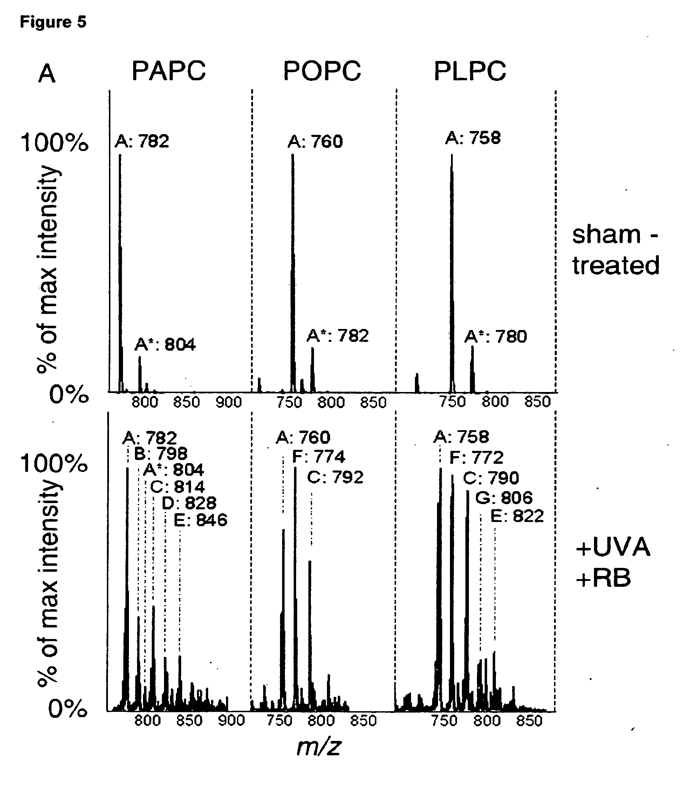

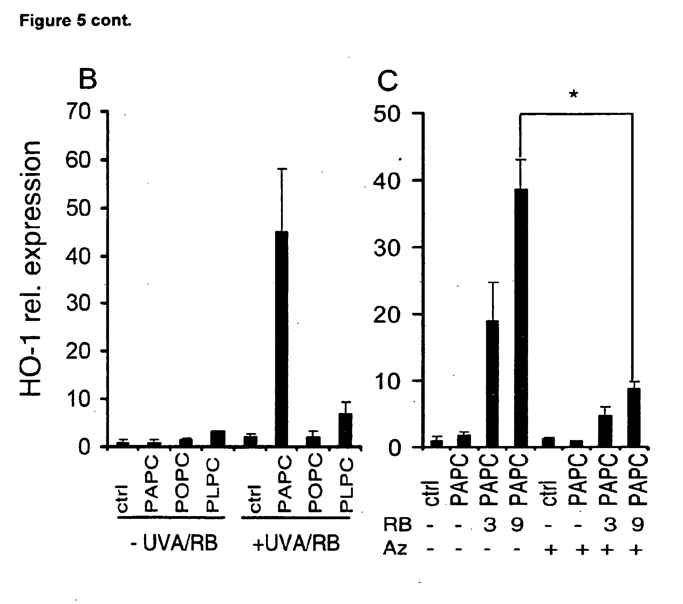

- **Figure 5:**: **UVA-1 irradiation of common phosphatidylcholine based phospholipids in the presence of the ¹O₂ generating dye Rose Bengal (RB) strongly promotes formation of long chain oxidation products**
(A) ESI-MS analysis of the sham-irradiated mother compounds PAPC, POPC and PLPC and ESI-MS analysis of PAPC, POPC and PLPC after irradiation with 10J/cm² UVA-1 in the presence of 20µM Rose Bengal (RB). Oxidation products were tentatively identified as: A - the [MH]+ molecular ion; A* - the [MNa]+ molecular ion; B - (-OH); C - (-OOH); D - PEIPC; E - di (-OOH); F - (=O); G - (-OH -OOH) of the corresponding mother compound.
(B) UVA-1/RB irradiated PAPC but not POPC and PLPC induce Heme Oxygenase 1 (HO-1) mRNA expression relative to non-treated control cells (ctrl). PAPC, POPC and PLPC not irradiated with UVA-1 in the presence of RB did not induce Heme Oxygenase 1 (HO-1) mRNA expression relative non-treated control cells (ctrl).
(C) UVA-1/RB irradiated PAPC induces Heme Oxygenase 1 (HO-1) mRNA expression relative to non-treated control cells to a lesser extent when sodium azide is present as compared to UVA-1/RB irradiated PAPC in the absence of sodium azide.
Relative expression of HO-1 mRNA [fold induction over control] was determined using quantitative real-time PCR (qPCR) normalized to expression of the housekeeping gene beta-2-microglobulin (B2M). Data represent means +/- S.D.

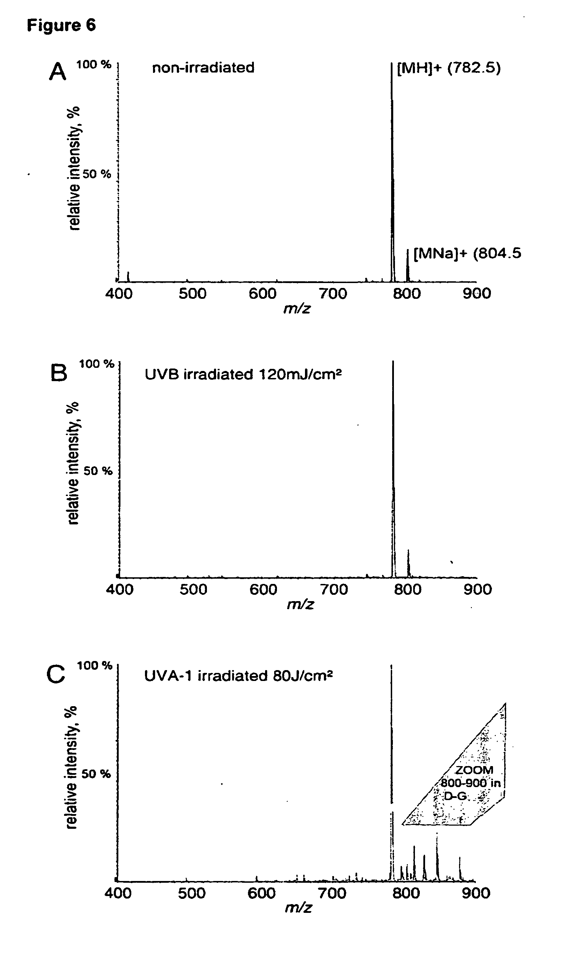

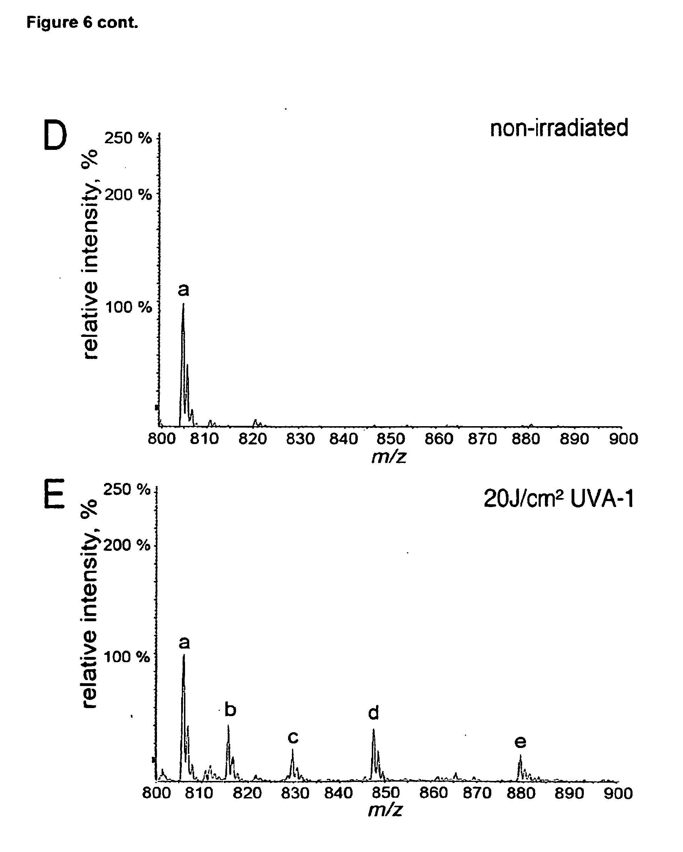

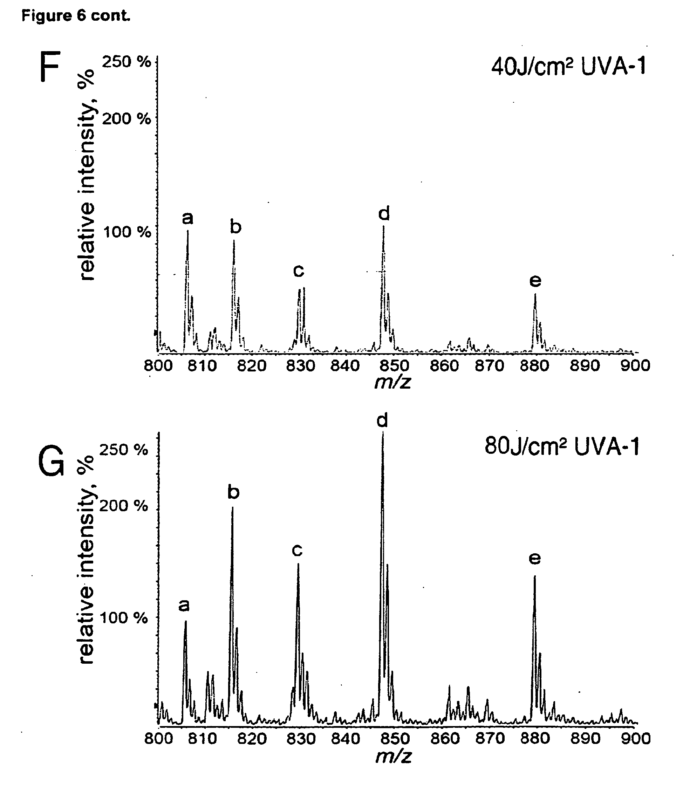

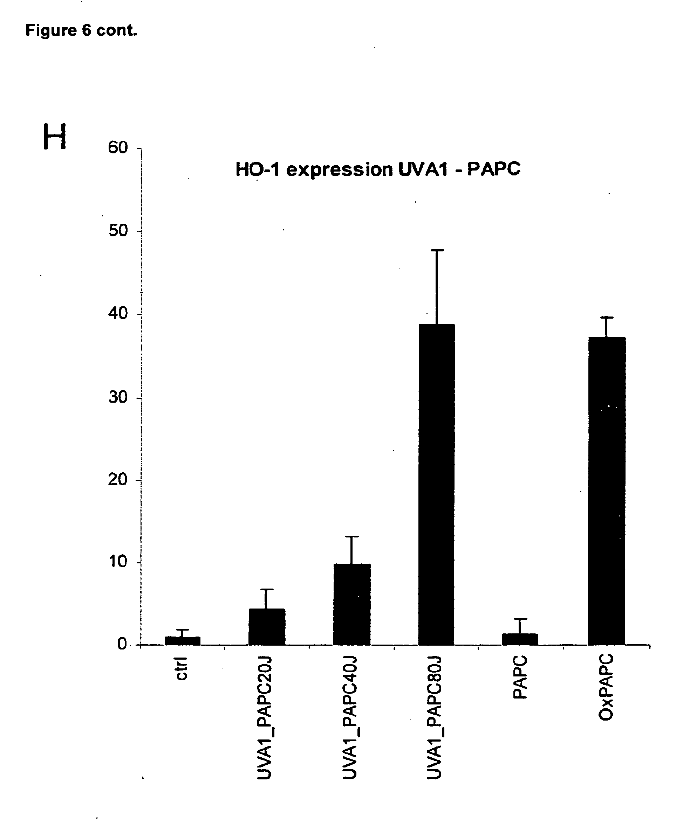

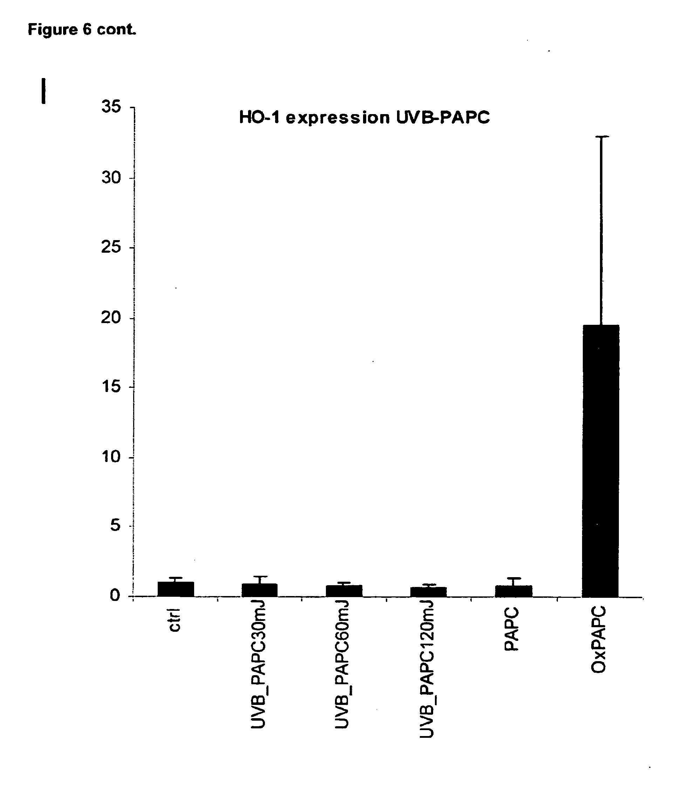

- **Figure 6:**: **UVA-1 but not UVB irradiation of PAPC leads to the generation of long chain oxidation products**
(A)-(G) Shown is the formation of mono-, di- and tri-hydroperoxides of the arachidonic acid moiety of PAPC (m/z ratio 814 [b], 846 [d], 878 [e]) as well as ions with the m/z ratio of 828 [c], relative to the [MNa]+ ion [a].
Generation of these oxidation products was UVA-1 fluency-dependent, while irradiation with corresponding fluencies of UVB (up to 120mJ/cm²) did not lead to formation of these oxidation products. The ion [c] with m/z 828,8 corresponds to 1-palmitoyl-2-(epoxy-isoprostane-E2)-sn-glycero-3-phosphorylcholine (PEIPC). Ions [b], [d] and [e] correspond to the mono- di- and tri-hydroperoxides of PAPC.
**Induction of Heme Oxygenase mRNA expression by UVA-1 treated PAPC**
(H) UVA-1 treated PAPC (UVA1_PAPC20J, UVA1_PAPC40J, UVA1_PAPC80J) but not untreated PAPC induce Heme Oxygenase 1 (HO-1) mRNA expression relative to autoxidized PAPC (oxPAPC) or non-treated control cells (cntrl). UVA1_PAPC20J was treated with UVA-1 until a fluency of 20 J/cm² was reached; UVA1_PAPC40J and UVA1_PAPC80J were treated accordingly until a fluency of 40 J/cm² respectively 80 J/cm² was reached.
(I) UVB treated PAPC (UVB_PAPC30mJ, UVB-PAPC60mJ, UVB_PAPC120mJ) but not untreated PAPC induce Heme Oxygenase 1 (HO-1) mRNA expression relative to autoxidized PAPC (oxPAPC) or non-treated control cells (cntrl). UVB_PAPC30mJ was treated with UVB until a fluency of 30 mJ/cm² was reached; UVB_PAPC60mJ and UVB_PAPC120mJ were treated accordingly until a fluency of 60 mJ/cm² respectively 120 mJ/cm² was reached.
Relative expression of HO-1 mRNA [fold induction over control] was determined using quantitative real-time PCR (qPCR) normalized to expression of the housekeeping gene beta-2-microglobulin (B2M). Data represent means +/- S.D.
- **Figure 7:**: UVA-1 treated PAPC (UVPAPC) but not untreated PAPC induces Heme Oxygenase 1 (HO-1) mRNA expression relative to non-treated control cells (ctrl) in primary dermal fibroblasts (FB) 4h after treatment with 100 µg/ml of UVPAPC or PAPC. Relative expression of HO-1 mRNA [fold induction over control] was determined using quantitative real-time PCR (qPCR) normalized to expression of the housekeeping gene beta-2-microglobulin (B2M). Data represent means +/- S.D. UVPAPC was treated with UVA-1 until a fluency of 40 J/cm² was reached.
- **Figure 8:**: **The figure shows a scheme of the procedure of activating extracts with UV light.**
- **Figure 9:**: **Thin layer chromatography of UVA-1 treated *Physcomitrella patens* extracts**
Shown is a thin layer chromatography of UVA-1 treated (right column) and untreated (left column) *Physcomitrella patens* extracts that were used in experiments on induction of Heme Oxygenase 1 (HO-1) expression wherein they exhibited an activity to induce HO-1 expression in keratinocytes.
- **Figure 10:**: **UVA-1 treatment of lipid extract reduces relative abundance of PAPC**
Shown is ESI-MS (positive mode 100-1000 da) analysis of a lipid extract before UVA-1 treatment (17A) and after UVA-1 treatment (17B). Photooxidaton using UVA-1 treatment in accordance with this invention reduces the relative abundance PAPC.
- **Figure 11:**: **Induction of Heme Oxygenase 1 mRNA expression in dermal fibroblasts by UVA-1 treated extracts from *Physcomitrella patens***
UVA-1 treated (UV_PE) and to a lesser extent UVA-1 treated PAPC (UV_PAPC) but not untreated *Physcomitrella patens* extract (PE) or untreated PAPC (PAPC) induce Heme Oxygenase 1 (HO-1) mRNA expression in dermal fibroblasts 4h after treatment with 100 µg/ml of lipids or extract relative to non-treated control cells (ctrl). Olive oil, UVA-1 treated olive oil, "Kern"oil (derived from *Cucurbita pepo L. var. styriaca I.*), UV-1 treated "kern"oil, corn oil or UVA-1 treated corn oil did not increase Heme Oxygenase mRNA expression.
Relative expression of HO-1 mRNA [fold induction over control] was determined using quantitative real-time PCR (qPCR) normalized to expression of the housekeeping gene beta-2-microglobulin (B2M). Data represent means +/- S.D.

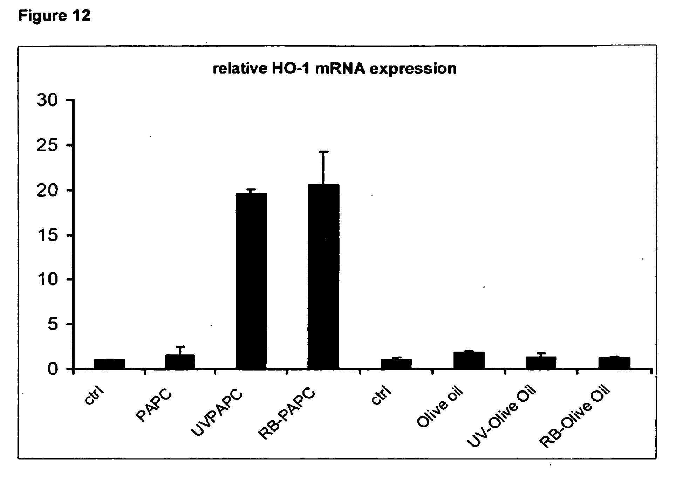

- **Figure 12:**: HO-1 mRNA expression in dermal fibroblasts 4h after stimulation with 100 µg/ml of either untreated PAPC ("PAPC"), untreated olive oil ("olive oil"), PAPC or olive oil treated with UVA-1 (40 J/cm²) ("UVPAPC" and "UV-Olive Oil", respectively) and PAPC or olive oil treated with UVA-1 (10J/cm²) in the presence of 90 µM,Rose Bengal ("RB-PAPC" and "RB-Olive Oil) relative to non-treated control cells (ctrl). Relative expression of HO-1 mRNA [fold induction over control] was determined using quantitative real-time PCR (qPCR) normalized to expression of the housekeeping gene beta-2-microglobulin (B2M). Data represent means +/- S.D.

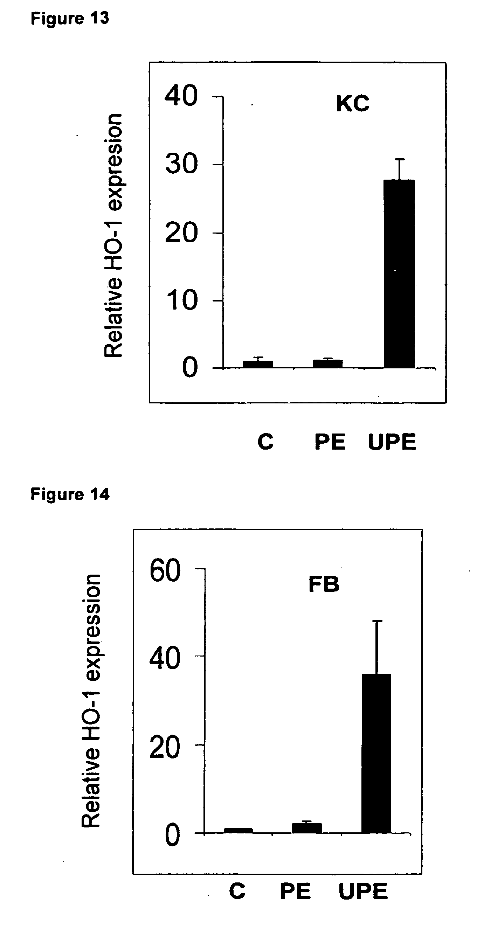

- **Figure 13:**: **Induction of Heme Oxygenase 1 mRNA expression in keratinocytes by UVA-1 treated extracts from *Physcomitrella patens***
UVA-1 treated (UPE) but not untreated (PE) *Physcomitrella patens* extracts induce Heme Oxygenase 1 (HO-1) mRNA expression relative to control levels (C) in primary keratinocytes (KC). Relative expression of HO-1 mRNA [fold induction over control] was determined using quantitative real-time PCR (qPCR) normalized to expression of the housekeeping gene beta-2-microglobulin (B2M). Data represent means +/- S.D.
- **Figure 14:**: **Induction of Heme Oxygenase 1 mRNA expression in dermal fibroblasts by UVA-1 treated extracts from *Physcomitrella patens***
UVA-1 treated (UPE) but not untreated (PE) *Physcomitrella patens* extracts induce Heme Oxygenase 1 (HO-1) mRNA expression relative to non-treated control cells (C) in primary dermal fibroblasts (FB). Relative expression of HO-1 mRNA [fold induction over control] was determined using quantitative real-time PCR (qPCR) normalized to expression of the housekeeping gene beta-2-microglobulin (B2M). Data represent means +/- S.D.

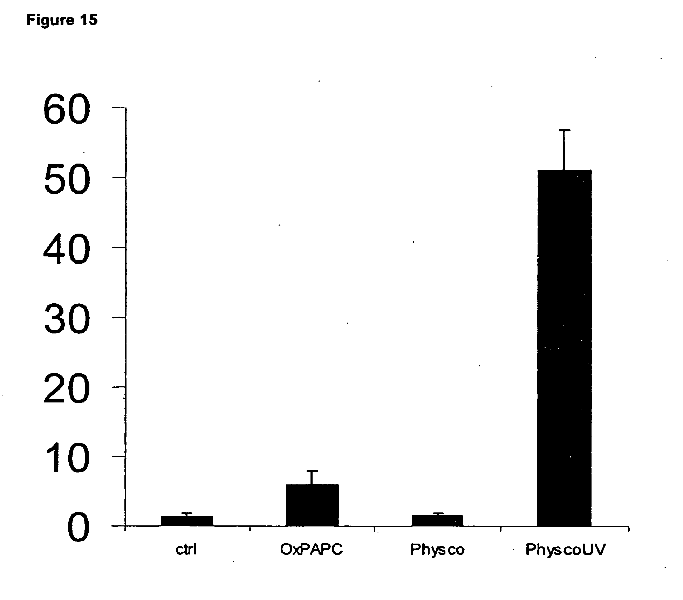

- **Figure 15:**: **Induction of Heme Oxygenase 1 mRNA expression in keratinocytes by UVA-1 treated extracts from *Physcomitrella patens***
UVA-1 treated (PhyscoUV) but not untreated (Physco) *Physcomitrella patens* extracts induce Heme Oxygenase 1 (HO-1) mRNA expression in keratinocytes 4h after treatment relative to cells treated with autoxidized PAPC (oxPAPC) or non-treated control cells (C). Relative expression of HO-1 mRNA [fold induction over control] was determined using quantitative real-time PCR (qPCR) normalized to expression of the housekeeping gene beta-2-microglobulin (B2M). Data represent means +/- S.D.

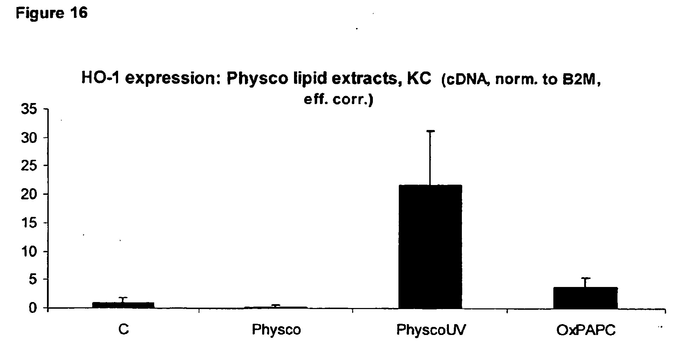

- **Figure 16:**: **Induction of Heme Oxygenase 1 mRNA expression in keratinocytes by UVA-1 treated extracts from *Physcomitrella patens***
UVA-1 treated (PhyscoUV) but not untreated (Physco) *Physcomitrella patens* extracts induce Heme Oxygenase 1 (HO-1) mRNA expression in keratinocytes 4h after treatment relative to cells treated with autoxidized PAPC (oxPAPC) or non-treated control cells (C). Relative expression of HO-1 mRNA [fold induction over control] was determined using quantitative real-time PCR (qPCR) normalized to expression of the housekeeping gene beta-2-microglobulin (B2M). Data represent means +/- S.D.

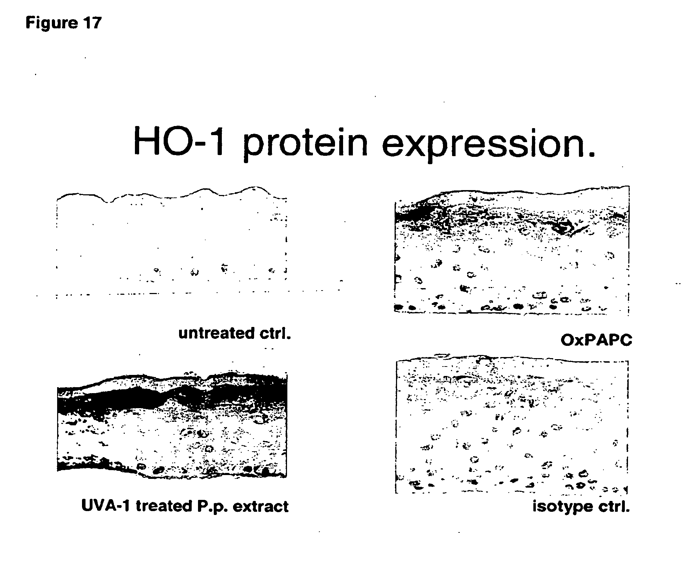

- **Figure 17:**: **Induction of Heme Oxygenase 1 protein expression in keratinocytes by UVA-1 treated extracts from *Physcomitrella patens***
UVA-1 treated *Physcomitrella patens* extract induces Heme Oxygenase 1 (HO-1) protein expression in skin equivalents relative to skin equivalents treated with autoxidized PAPC (oxPAPC), isotype (isotype ctrl) or non-treated skin equivalents (untreated ctrl). Skin equivalents were treated for 18 h with 100 mg/ml lipids or extract.

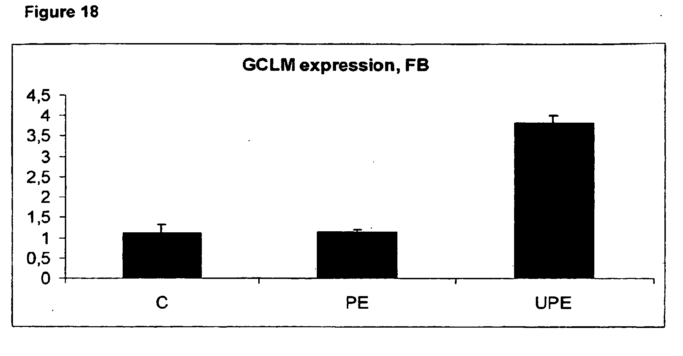

- **Figure 18:**: **Induction of Glutathione Cysteine Ligase Modifier subunit (GCLM) mRNA expression in dermal fibroblasts by UVA-1 treated extracts from *Physcomitrella patens***
UVA-1 treated (UPE) but not untreated (PE) *Physcomitrella patens* extracts induce Glutathione Cysteine Ligase Modifier subunit (GCLM) mRNA expression relative to non-treated control cells (C) in primary dermal fibroblasts (FB). GCLM is a rate limiting enzyme for glutathione (GSH) synthesis. Relative expression of GCLM mRNA [fold induction over control] was determined using quantitative real-time PCR (qPCR) normalized to expression of the housekeeping gene beta-2-microglobulin (B2M). Data represent means +/- S.D.

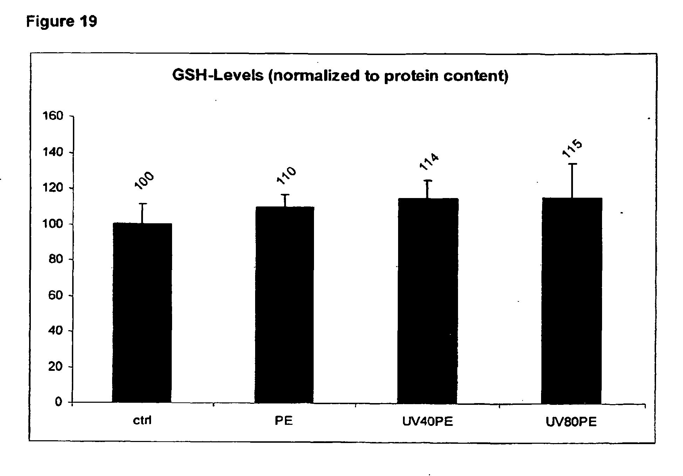

- **Figure 19:**: **Increase of cellular glutathione levels in dermal fibroblasts by UVA-1 treated extracts from *Physcomitrella patens***
UVA-1 treated (UV40PE, UV80PE) but not untreated (PE) *Physcomitrella patens* extract leads to an increase of cellular glutathione (GSH) levels in dermal Fibroblasts 18h after treatment relative to non-treated control cells (ctrl). UV40PE was treated with UVA-1 until a fluency of 40 J/cm² was reached, UV80PE was treated accordingly until a fluency of 80 J/cm² was reached.

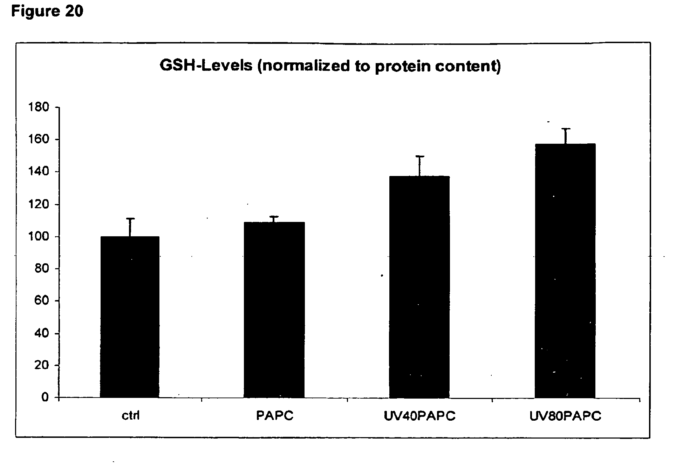

- **Figure 20:**: **Increase of cellular glutathione levels in dermal fibroblasts by UVA-1 treated PAPC**
UVA-1 treated PAPC (UV40PAPC, UV80PAPC) leads to an increase of cellular glutathione (GSH) levels in dermal Fibroblasts 18h after treatment relative to non-treated control cells (ctrl). UV40PAPC was treated with UVA-1 until a fluency of 40 J/cm² was reached, UV80PAPC was treated accordingly until a fluency of 80 J/cm² was reached.

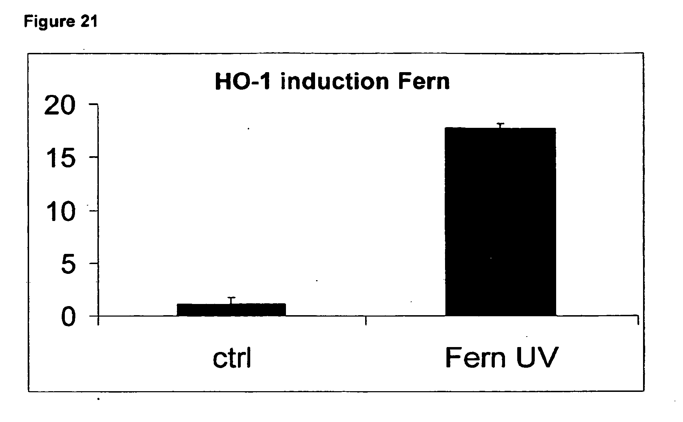

- **Figure 21:**: **Induction of Heme Oxygenase 1 mRNA expression in dermal fibroblasts by UVA-1 treated extracts from fern (*Thelypteris noveboracensis*)**
HO-1 mRNA expression in dermal fibroblasts 4h after stimulation with UVA-1 (40J/cm2) irradiated lipid extract derived from the fern *Thelypteris noveboracensis* ("Fern UV") relative to non-treated control cells (ctrl). Relative expression of HO-1 mRNA [fold induction over control] was determined using quantitative real-time PCR (qPCR) normalized to expression of the housekeeping gene beta-2-microglobulin (B2M). Data represent means +/- S.D.

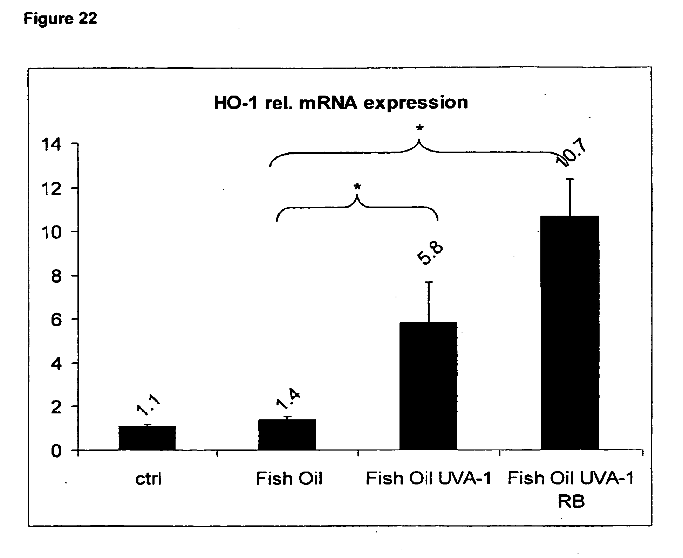

- **Figure 22:**: **Induction of Heme Oxygenase 1 mRNA expression in dermal fibroblasts by UVA-1 treated extracts from fish (fish oil)**
HO-1 mRNA expression in dermal fibroblasts 4h after stimulation with 5mg/ml untreated ("fish oil"), UVA-1 (40J/cm2) irradiated ("Fish oil UVA-1) and Rose Bengal - treated plus UVA-1 (10J/cm2) irradiated fish oil ("Fish oil UVA-1 RB") relative to non-treated control cells (ctrl). Relative expression of HO-1 mRNA [fold induction over control] was determined using quantitative real-time PCR (qPCR) and normalized to expression of the housekeeping gene beta-2-microglobulin (B2M). Data represent means +/- S.D, * indicates p<0.05 in a 2-tailed Student's t-test.

The Examples illustrate the invention.

### Example 1:

### Photooxidation generates biologically active phospholipids that induce heme oxygenase-1 in skin cells

### Material and methods

### Cell Culture

Neonatal Human Epidermal Keratinocytes (KC) derived from foreskin are obtained from Clonetics (San Diego, CA). Keratinocytes are cultured in keratinocyte growth medium (KGM) up to the fifth passage. Human neonatal skin fibroblasts (FB) are obtained from Cascade Biologics (Portland, OR) and grown in Dulbecco's modified Eagle's medium, Gibco (Gaithersburg, MD) supplemented with 10% fetal calf serum (FCS) and penicillin/ streptomycin (1000U per ml, Gibco) to sub-confluence.

### Skin equivalents (SE)

*In vitro*-reconstructed skin equivalents are generated as described previously (Rendl, J.Invest Dermatol. 119, 1150-1155 (2002)). Briefly, 1.3 x 10⁶ KCs are added on top of a collagen gel containing fibroblasts. After overnight incubation, the medium from the upper chamber is removed, thus putting the KC at air-liquid interface. Afterward, SEs are cultured in serum-free KC-defined medium, which is KC growth medium without bovine pituitary extract, supplemented with 1.3 mM calcium, 10 µg/ml transferrin, 50 µg/ml ascorbic acid, and 0.1% BSA.

### Quantitative real-time PCR (qPCR)

RNA is isolated using Trizol reagent (Invitrogen). Nine hundred ng of total RNA are reverse-transcribed with murine leukemia virus reverse transcriptase (MuLV, Applied Biosystems, Foster City, CA) and oligo(dT) primers. The following forward (F) and reverse (R) primers are used: HO-1: F, 5'-AAGATTGCCCAGAAAGCCCTGGAC-3'; R, 5'-AACTGTCGCCACCAGAAAGCTGAG-3'; B2M; F 5'-GATGAGTATGCCTGCCGTGTG-3'R,5'-CAATCCAAATGCGGCATCT-3' qPCR is performed using LightCycler technology and the Fast Start SYBR Green I kit (both Roche Applied Science, Basel, Switzerland). In all assays, cDNA is amplified using a standardized program (10-min denaturing step; 55 cycles of 5 s at 95 °C, 15 s at 65 °C, and 15 s at 72 °C; melting point analysis in 0.1 °C steps). Quantification of target gene expression is performed using a mathematical model by Pfaffl (Pfaffl, Nucleic Acids Res. 29, e45 (2001)). The expression of the target molecule is normalized to the expression of β-2 microglobulin.

### Western Blot Analysis

Bound HO-1 antibodies (SPA-896, Stressgen, Victoria, Canada) are detected by anti-IgG conjugated with peroxidase and subsequent chemiluminescent detection.

### UV-irradiation

UV-irradiation is carried out as described previously (Mildner, Photochem.Photobiol. 70, 674-679(1999)). As a light source for UVA-1 a Mutzhas Supersun 5000-type solar simulator (Mutzhas, Munich, Germany) filtered for the emission of UVA-1 (340-390 nm) is used. UVB (280-320 nm) irradiation is performed with a Waldmann F15 T8 tube (Waldmann, Villingen, Germany). Cells are irradiated with 10 to 40 J/cm² of UVA-1 or with 20 mJ/cm2 of UVB under a thin layer of PBS at 25°C.

The maximal amount of UVA-1 radiation used in this study (40 J/cm²) would reach the surface of the skin during 112 min of sunlight exposure at noon at a northern latitude of 35°; 30% would reach the dermis (Vile, Free Radic.Biol.Med. 18, 721-730 (1995)). This is within the fluency range used for UVA-1 phototherapy (Mang, Photodermatol.Photoimmunol.Photomed. 21, 103-108 (2005)).

### Immunofluorescence / Immunohistochemistry

Dermal fibroblasts are grown on 8-well permanox slides (Nalgene, Rochester, NY). Cells are fixed with 80% methanol (5 min, 4°C) and labeled with EO6 (kindly provided by J.L. Witztum, San Diego, CA) and control IgM antibody as described in (Chang, Proc.Natl.Acad.Sci.U.S.A 96, 6353-6358 (1999)). Double staining is done using additionally 0.5 mg/ml anti-Caspase 3 rabbit IgG antibody (R+D Systems, Minneapolis, MN) and control IgG.

Formalin fixed paraffin sections of SE are stained with HO-1 antibodies (SPA-896, Stressgen, Victoria, Canada).

### Lipid oxidation

PAPC is oxidized by exposure of the dry lipid to air for 72 h to generate OxPAPC. PAPC dried to a thin film on a glass support is irradiated with UVA-1 with 160 J/cm² to generate UV-PAPC. For treatment with singlet oxygen all lipids are vortexed in PBS containing 20µM Rose Bengal and irradiated with 10 J/cm² UVA-1 (Fig.5 *A,B*) or under a commercial 35W halogen lamp at a distance of 30 cm for 30 minutes (Fig.5C). The extent of oxidation was monitored by ESI-MS as described previously (Watson, J.Biol.Chem. 272, 13597-13607 (1997)). The UVA-1 irradiated lipids are solubilized, emulsified or suspended in the respective growth medium before skin cells or the skin equivalent are treated.

### Thin-layer chromatography

TLC analysis of lipids is performed on Silica gel 60 TLC plates (Merck, Austria) using a mixture of chloroform-methanol-water (100:50:10 v/v/v) as a developing solvent. Lipid spots are visualized after treatment with 10% copper sulfate in an 8.5% aqueous solution of ortho-phosphoric acid and subsequent heating at 180°C.

### Mass spectrometry

Mass spectrometry is performed on a PESciex API 365 triple quadrupole mass spectrometer (Applied Biosystems, Foster City, CA, USA) equipped with an electrospray source. Flow injection experiments are performed by injecting 20 µl-aliquots of lipid samples dissolved in 200 µl of methanol-water-formic acid (80:20:0.1, v/v/v) into a stream of the same solvent mixture, delivered by an HPLC system (HP1100, Agilent Technologies, Waldbronn, Germany). Spectra are acquired in the positive mode in the range of 400-900 Da. LC-MS and LC-MS/MS is performed using an Agilent Zorbax Eclipse XDB-C8 column (150x4.6 mm, 5 µm). Samples are dissolved in mobile phase A (10 mM ammonium acetate in methanol-water, 80:20, v/v). Analytes are eluted using a gradient from 25% mobile phase B (10 mM ammonium acetate in methanol) to 100% B in 30 minutes, followed by an isocratic step at 100% B for 30 minutes. MS and MS/MS detection is performed in the scan mode (400-900 Da) or the multiple reaction monitoring mode detecting the phosphatidylcholine-specific fragment at 184.1 Da produced from various precursor ions (594.5, 610.6, 782.7, 814.8, 828.8, 846.8, 878.8 Da) at a collision energy of 35 eV.

### Results

### OxPAPC induces HO-1 expression in keratinocytes (KC) and dermal fibroblasts (FB)

Recently, it has been shown that HO-1 expression in vascular endothelial and smooth muscle cells is strongly upregulated by OxPAPC, which had been generated by air exposure (Ishikawa, J.Clin.Invest 100, 1209-1216 (1997), Kadl, Vascul.Pharmacol. 38, 219-227(2002), Kronke, J.Biol.Chem. 278, 51006-51014 (2003)). It was examined whether OxPAPC has similar effects on skin cells. As has been reported previously (Basu-Modak, Free Radic.Biol.Med. 20, 887-897(1996)), treatment of FB with UVA-1 (40 J/cm²), but not UVB (20 mJ/cm²), leads to a strong induction of HO-1 mRNA expression, as did addition of OxPAPC (100 µg/ml) (Fig 1*A*). When OxPAPC was added at different concentrations to cultures of primary human epidermal KC and FB, HO-1 mRNA and protein expression were strongly induced in both cell types in a dose-dependent manner (Fig. 1*B,C*). Strong induction of HO-1 mRNA and protein was also observed when instead of KC in monolayer cultures, epidermal equivalent cultures which mimic more closely the in vivo situation (Rendl, J.Invest Dermatol. 119, 1150-1155 (2002)) were exposed to OxPAPC for 4 consecutive days (Fig. 1*D*). HO-1 protein expression was most pronounced in the epidermal layers immediately below the stratum corneum. These data demonstrate that like UVA-1, OxPAPC is able to strongly stimulate HO-1 expression in skin cells.

### HO-1-inducing activity is contained in long-chain, but not fragmented, oxidized phospholipids

OxPAPC contains several oxidation products of PAPC that can roughly be classified into products that result from insertion of oxygen into the *sn-*2 chain on the one hand, and the chain-shortened products on the other hand. Distinct biological properties have been attributed to compounds of each class (Leitinger, Proc.Natl.Acad.Sci.U.S.A 96, 12010-12015 (1999), Subbanagounder, Free Radic.Biol.Med. 28, 1751-1761 (2000). To identify the compounds within the OxPAPC preparation responsible for HO-1 induction long-chain oxidation products (LC), short-chain oxidation products (SC), and polar lipids (PO) prepared by thin layer chromatography (TLC) were separately tested. As shown in Figure 2A HO-1 was strongly induced in fibroblasts by the long-chain oxidized fraction of phospholipids while the other fractions had virtually no effect. These data were confirmed when the synthetic short chain compounds 1- palmitoyl-2-(5-oxovaleroyl)-*sn*-glycero-3-phosphorylcholine (POVPC), 1-palmitoyl-2-glutaroyl-*sn*-glycero-3-phosphorylcholine (PGPC) and 1-palmitoyl-2-lyso-*sn*-glycero-3-phosphorylcholine (LysoPC) failed to induce HO-1 expression as did unoxidized PAPC and di-myristoyl-phosphorylcholine (DMPC) (Fig. 2*B*). This data demonstrate that the capacity to induce HO-1 resides within the long-chain oxidation products of PAPC. In order to examine the possible contribution of lipid hydroperoxides, which are present in the OxPAPC preparation, to the induction of HO-1 expression, fibroblasts were incubated with the ROS scavengers β-carotene, vitamin C, vitamin E, and sodium azide prior to exposure to OxPAPC. This treatment did not significantly reduce OxPAPC-mediated HO-1 induction. Furthermore, treatment of OxPAPC with triphenylphosphine (TPP), which reduces lipid hydroperoxides (Mihaljevic, Free Radic.Biol.Med. 21, 53-63, (1996)) only slightly reduced the capacity of OxPAPC to induce HO-1 expression (not shown). These data demonstrate that neither peroxidation of host lipids caused by OxPAPC nor hydroperoxide groups present in OxPAPC are responsible for HO-1 induction.

### UVA-1 irradiation of PAPC (UV-PAPC) leads to formation of long-chain oxidation products that induce HO-1 expression

PAPC is abundant among phospholipids of cellular membranes (Williams, J.Lipid Res. 41, 1585-1595 (2000)). Since it contains a polyunsaturated fatty acyl side chain (20:4) at the *sn*-2 position it is prone to oxidation by UV light, singlet oxygen or free radicals (McIntyre, J.Biol.Chem. 274, 25189-25192 (1999), Girotti, J.Photochem.Photobiol.B 63, 103-113 (2001). For the skin, UVA irradiation is an important inducer of oxidative stress and results in the expression of HO-1 in fibroblasts (Keyse, Proc.Natl.Acad.Sci.U.S.A 86, 99-103. (1989)). To investigate the direct effects of UVA-1 irradiation on PAPC oxidation synthetic PAPC was irradiated in vitro. PAPC was dried to a film on a glass support and irradiated with 160 J/cm² UVA-1. The irradiated PAPC (UV-PAPC) and the sham treated PAPC were analyzed by electrospray ionisation mass spectrometry (ESI-MS). After UVA-1 irradiation mono-, di- and tri-hydroperoxides of the arachidonic acid moiety of PAPC (*m*/*z* ratio 814.7, 846.7, 878.7) as well as molecules with the *m*/*z* ratio of 828.8, corresponding to 1-palmitoyl-2-(epoxyisoprostane E2)-*sn*-glycero-3-phosphorylcholine (PEIPC) were formed (Figure 3*A,B*). PEIPC, a strong inducer of HO-1 expression (Ishikawa, J.Clin.Invest 100, 1209-1216 (1997)), was also present in the active TLC-fraction labeled "LC" in Figure 2*A*, as evidenced by ESI-MS and recently described by us (Birukov, Circ.Res. 95, 892-901 (2004)).

In order to investigate the respective biologic activities, sham treated PAPC and UV-PAPC were added to cultures of FB and HO-1 mRNA expression was measured after 4 hours. UV-PAPC, but not sham treated PAPC, strongly induced HO-1 expression (Fig. 3C) confirming that UVA-1-mediated oxidation of PAPC leads to the formation of biologically active lipid mediators.

### UVA-1 irradiation generates oxidation-specific phospholipid epitopes in human fibroblasts

To investigate whether UVA-1 also leads to phospholipid oxidation in living cells, FB were irradiated and analyzed at different time points for the presence of PAPC oxidation products. The murine monoclonal IgM antibody EO6 binds to oxidized phosphorylcholine-containing phospholipids present in OxPAPC, oxidized LDL and in the cell membranes of apoptotic cells. For instance, EO6 recognizes POVPC and PEIPC, but not Lyso-PC (Subbanagounder, J.Biol.Chem. 277, 7271-7281 (2002), Palinski, J.Clin.Invest 98, 800-814 (1996), Horkko, J.Clin.Invest 103, 117-128 (1999)). Irradiation of FB with UVA-1 resulted in a dose-dependent increase of intracellular EO6 immunoreactivity, demonstrating the formation of phospholipid oxidation products within 10 minutes after UVA-1 exposure (Fig. 4*A-D*). Immunostaining of cells 18 hours after irradiation revealed a similar staining with EO6 antibodies. Double-staining with antibodies detecting active Caspase 3, showed that EO6 immunoreactivity was not confined to apoptotic cells (EO6 positive: 45.5 %, aC-3 positive: 15.6%, double positive: 8.5% after irradiation with 60J/cm²) (Fig. 4*E*-*H*). At lower fluencies of UVA-1, apoptosis (as detected with aC-3) in FB did not occur at all (Fig. 4*F*), whereas EO6 immunoreactivity was found on 12% of cells as compared to 3% of sham-treated cells. These data demonstrate for the first time that UVA-1 irradiation is able to induce formation of oxidized phospholipids in cells that remain viable. When lipids were extracted from dermal fibroblasts immediately after UVA-1 irradiation and compared to extracts from sham irradiated cells, we observed accumulation of molecules with the retention times and *m*/*z* ratio (828.8) of PEIPC, using LC-MS/MS analysis (not shown), further supporting the finding that PEIPC can form in cells *in vivo.*

### Generation of HO-1-inducing oxidized phospholipids by UVA-1 involves singlet oxygen and requires a sn-2 arachidonate moiety

Singlet oxygen (¹O₂) is an important ROS that mediates photooxidation by UVA-1 (Ryter, Free Radic.Biol.Med. 24, 1520-1534 (1998)). In order to investigate the role of singlet oxygen in UVA-1-mediated oxidation of PAPC, the ¹O₂ generator Rose Bengal (RB) was included during irradiation. Subsequently, it was analyzed whether generation of ¹O₂ would enhance the formation of lipid oxidation products and their biological effects at fluencies as low as 10 J/cm². MS analysis showed that hydroperoxides and PEIPC were generated from PAPC by this treatment (Fig. 5A upper left and lower left panels). POPC and PLPC, two other abundant membrane phospholipids, differ from PAPC in their length and number of double bonds of the *sn*-2 acyl chain; a single double bond in 1-palmitoyl-2-oleoyl-*sn*-3-glycerophosphorylcholine (POPC) (18:1) and two double bonds in 1-palmitoyl-2-linoleyl-*sn*-3-glycerophosphorylcholine (PLPC) (18:2) allow oxidative formation of hydroperoxides but not of the epoxyisoprostanoid oxidation product found in PAPC (20:4). Accordingly, irradiation of POPC and PLPC, in the presence of RB, while leading to the formation of hydroperoxides, did not result in the formation of comparable epoxyisoprostane structures (Fig. 5A, middle and right panels). Comparison of oxidation products derived from various phospholipids demonstrated that only PAPC but not POPC or PLPC that had been irradiated in the presence of RB, efficiently induced expression of HO-1 in FB (Fig. 5*B*). A further indication for the involvement of ¹O₂ in biological processes is that its action can be quenched by sodium azide. When PAPC was irradiated with white light from a commercial 35W source at a distance of 30 cm for 30 minutes in the presence of RB (3 and 9 µM), addition of sodium azide (10mM) prevented the generation of oxidation products that were able to induce HO-1 mRNA expression in FB (Fig. 5C).

These findings demonstrate that the presence of ¹O₂ facilitates the formation of long chain oxidation products of phospholipids and the resulting capacity of some of these products, such as PAPC-derived PEIPC, to induce HO-1 expression.

In accordance with this invention it could be shown that UVA-1 irradiation dose-dependently and directly oxidizes the common membrane phospholipid PAPC, leading to formation of biologically active compounds capable of up-regulating the protective HO-1 expression in skin cells. these compounds were identified as epoxyisoprostane-containing phosphatidylcholine (PEIPC). Furthermore, it was shown that the oxidation of PAPC by UVA-1 depends on the generation of singlet oxygen. Biologically active oxidized phospholipids generated by UVA-1 may thus contribute to the effect of UVA-1 irradiation on normal and diseased skin. These lipid mediators might be able to mimic the beneficial effects of UVA-1 in therapy of skin diseases while avoiding the potential long term disadvantages of repeated UVA-1 irradiation. Accordingly, the use of the herein identified compounds and "UVA-1 activated" extracts (see following examples and embodiments disclosed in the present invention) are medically as well as cosmetically useful in pharmaceutical compositions and in form of cosmetic compositions. The corresponding uses are evident for the person skilled in the art and comprise in particular uses which are also described and known in the art for UVA-light treatments (in the medical field or cosmetic UVA-light applications. The present invention provides for means and methods for the development and use of novel and inventive compounds, i.e. UVA-1 activated extracts derived from organic material (which comprises originally (poly) unsaturated fatty acids or groups derived from a (poly) unsaturated fatty acid, preferably a PUFA comprising at least 4 double bonds). As pointed out above, these compounds are useful in a cosmetic setting but also in pharmaceutical and medical medications. Furthermore, such compounds, i.e. the here described UVA-1 activated extract from *Physcomitrella patens* may also be useful in pre-medical or preventive settings, for example in sun protection products, like sun creams, sun lotions, sun (lip) balm etc. Also, the extracts as described herein may be useful in cosmetics, like day- or night creams, anti-ageing creams, anti-ageing lotions, anti-ageing balms and the like. The following examples provide for embodiments of these kind of activated extracts and also provide for corresponding technical information how such extracts and, accordingly, the compounds of this invention can be obtained.

### Example 2:

### UVA-1 irradiation but not UVB irradiation produces biologically active phospholipids that induce heme oxygenase -1 mRNA expression in a dose-dependent manner in skin cells

### Material and methods

### Cells

Human neonatal skin fibroblasts (FB) are obtained from Cascade Biologies (Portland, OR) and grown in Dulbecco's modified Eagle's medium, Gibco (Gaithersburg, MD) supplemented with 10% fetal calf serum (FCS) and penicillin/ streptomycin (1000U per ml, Gibco) to sub-confluence.

### Lipids

1-palmitoyl-2-arachidonoyl-sn-glycero-3-phosphorylcholine (PAPC, Avanti Lipids)

### Autoxidized PAPC

Autoxidized PAPC (oxPAPC) is obtained according to the method described in example 1.

### UV-irradiation

UVA-1 irradiation of cells respectively PAPC is carried out as described in example 1 until a fluency of 20 J/cm², 40 J/cm² respectively 80 J/cm² is reached. UVB irradiation is carried out correspondingly until a fluency of 30 mJ/cm², 60 mJ/cm² respectively 120 mJ/cm² is reached.

### Treatment of fibroblasts with UV-irradiated extract

Fibroblasts are treated with autoxidized PAPC, UVA-1 treated PAPC or UVA-1 untreated PAPC (100 µg/ml) as described in Example 1. Total RNA is isolated after 4 hours from treated cells and untreated control cells.

### Quantitative real-time PCR (qPCR)

Relative expression of HO-1 mRNA in dermal fibroblasts is determined 4h after stimulation with lipids or extracts as specified above using quantitative real-time PCR (qPCR). Normalization is standardized by comparison to the expression of the housekeeping gene beta-2-microglobulin (B2M) as described in Example 1.

### Results

Figure 6 shows that treatment of PAPC with UVA-1 but not UVB leads to the generation of long chain oxidation products. While by irradiation with UVA-1 oxidation products of PAPC such as the mono-, di- and tri-hydroperoxides and PEIPC were produced, the irradiation with corresponding fluencies UVB did not generate any of these oxidation products. Furthermore, UVB treated PAPC was not able to induce Heme Oxygenase 1 mRNA expression in skin cells. On the contrary, UVA-1 treated PAPC induced HO-1 mRNA expression. The relative expression of HO-1 mRNA was comparable in cells treated with autoxidized PAPC and UVA-1 treated (80J/cm²) PAPC.

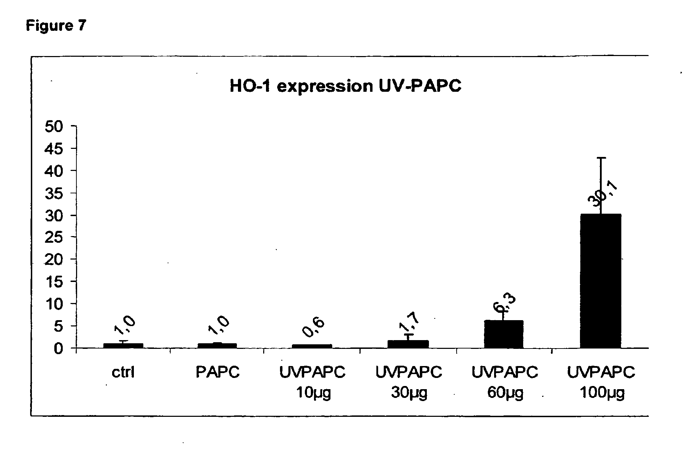

Figure 7 clearly shows the dose-dependent Heme Oxygenase 1 (HO-1) mRNA expression in primary dermal fibroblasts (FB) 4h after treatment with 10 µg/ml, 30 µg/ml, 60 µg/ml or 100 µg/ml of UVPAPC respectively relative to cells treated with PAPC or non-treated control cells. While an increase in mRNA expression can be observed after treatment with 30 µg/ml of UVPAPC a maximal HO-1 expression is induced, for example, after treatment with 100 µg/ml of UVPAPC.

### Example 3:

### Expression of various genes is induced by autoxidized PAPC and UVA-1 treated PAPC

### Material and methods

### Cells and Lipids

Cells and lipids are used as described in example 2.

### Autoxidized PAPC

Autoxidized PAPC (oxPAPC) is obtained according to the method described in example 1.

### UV-irradiation

UVA-1 irradiation of cells respectively PAPC is carried out as described in example 1 until a fluency of 40 J/cm² is reached.

### Treatment of fibroblasts with UV-irradiated extract

Fibroblasts are treated with autoxidized PAPC, UVA-1 treated PAPC or UVA-1 untreated PAPC (100 µg/ml) as described in Example 1. Total RNA is isolated 4h after stimulation with 100 µg/ml of lipids from treated cells and untreated control cells.

### Microarray Hybridization

Hybridization to human U133A Plus 2.0 GeneChips (Affymetrix, Santa Clara, CA) and scanning of the arrays was carried out according to manufacturer's protocols. The arrays were scanned using the GeneArray scanner (Affymetrix). Image analysis was performed with GeneChip software (Affymetrix, RMA). Normalization was performed by global scaling, with the arrays scaled to an average intensity of 500.

### Results

Previously, it has been shown that UVA-1 mediated photooxidation of the membrane phoshopolipid PAPC induces the expression of the stress response gene Heme Oxygenase 1 (HO-1). In order to investigate whether oxidized phospholipids regulate a larger number of stress response genes, dermal fibroblasts were treated with UVA-1 (40J/cm²), oxPAPC and UV-PAPC (100µg/ml each). In particular, the effect of the primary direct UVA-1 oxidation products of PAPC (UV-PAPC) which contain mainly the long-chain oxygenation products of the arachidonic acid moiety of PAPC was to be investigated.

Upon RNA isolation a microarray expression analysis was performed using the Affymetrix U133 2.0 plus system. Only genes that were up-or downregulated more than two-fold relative to untreated control cells were regarded. Genes regulated by unoxidized PAPC were ignored. 39 of the genes regulated by UVA-1 were coregulated by oxPAPC. 6 genes of this group were further coregulated by UV-PAPC comprising heme oxygenase-1 (HO-1), glutamate-cysteine ligase modifier subunit (GCLM), aldo-keto reductase family 1 member C1 (AKR1C1), aldo-keto reductase family 1 member C2 (AKR1C2), interleukin 8 (IL8) and a gene of unknown function (FLJ36701) by activation of the redox sensitive transcription factor nuclear factor (erythroid-derived 2)-like 2 (NFE2L2 alias Nrf2). While it has been clearly shown that HO-1 and GCLM play an important role in the antioxidant response such a role has not been confirmed for the remaining genes.

| UVA-1 | OxPAPC | UV-PAPC | **Genes** |
|---|---|---|---|
| ▲ | ▲ | ◄► | **39** |
| ▲ | ▲ | ▲ | **6** |

| | |
|---|---|
| HMOX1 | Heme oxygenase |
| IL8 | Cytokine |
| AKR1C1 | Oxidoreductase |
| AKR1C2 | Oxidoreductase |
| FLJ36701 | Hypothetical |
| GCLM | Glutathione synthesis |

### Discussion

HO-1 and GCLM are expressed at lower levels in aged tissues. Both genes contribute to the antioxidant defense and are protective against oxidative stress as induced by sunlight. HO-1, GCLM, IL8, ADR1C1, ADR1C2 and FLJ36701 are within the group of Phase II detoxification genes, which are regulated by the transcription factor Nrf2 (NFE2L2). Activation of these genes and the transcription factor may induce an increased degradation of dangerous and possibly carcinogenic substances that accumulate in tissues. Consequently, UVA-1 treated extracts derived from organic material comprising a high amount of polyunsaturated fatty acids or groups derived from a polyunsaturated fatty acid, preferably a PUFAs comprising at least 4 double bonds, in particular the herein exemplified (activated) extract from *Physcomitrella patens* will be able to prevent or delay ageing of cells, in particular skin cells or act as positive modulator in diseases or disorders or act as cancer chemopreventives or chemotherapeutics, e.g. in skin cancer and the like.

### Example 4:

### Obtaining, activating and characterizing an extract from organic material

### Extract from Physcomitrella patens

A lipid extract from Physcomitrella is obtained according to a modified Nichols' method of lipid extraction. Plant tissue is washed in phosphate buffered saline (PBS) wich is supplemented with 0.5 mM EDTA (pH 8.0). After centrifugation the supernatant is removed and the plant tissue is dried and weighed. Per gram plant material 100 g isopropanol corresponding to 127 ml isopropanol are added. Subsequently, the plant tissue is minced and macerated using a "Ultra Turrax". The mixture is then filtrated through a glass grain filter. In a subsequent step, the samples may be shortly (from 5 to 20 min) boiled in the organic solvent such as iso-propanol to avoid unwanted cleavage caused by lipases present in plants. These (phospho)lipases, especially phospholipase D, are activated by alcohols or diethyl ethers present in extraction mixtures and may be inactivated by boiling.

The dried filtrate is solubilised in chloroform/methanol (2:1 v/v). After evaporation of chloroform/methanol the material is solubilised again in 1 ml PBS. After adding 9 ml chloroform/methanol (2:1 v/v) and 1 µl Butylhydroxytoluol (final concentration 0,01 % BHT) the suspension is vortexed vigorously for 1 min and transferred on ice. After addition of 2.5 ml 0.7 M aqueous formic acid the suspension is vortexed or shaked vigorously again. The mixture is centrifuged (5 min, 1500 rpm) and the lower organic phase is transferred into a new tube. The organic solvents are removed either by drying under a nitrogen (argon) stream or by evaporating under reduced pressure. 0.3 - 0.5 ml methanol are added and the organic phase is removed under the nitrogen stream or by evaporating under reduced pressure. The previous step is repeated 1 - 2 times. Finally, the dried material is solubilised in chloroform.

Typically, a yield of 9.0 mg per 0.8 g centrifuged fresh material referring to the filtrate gained after the isopropanol extraction can be obtained, whereas the final yield after chloroform/methanol extraction is about 3.8 mg per 0.8 g centrifuged fresh material.

### Activation of an extract by UV light

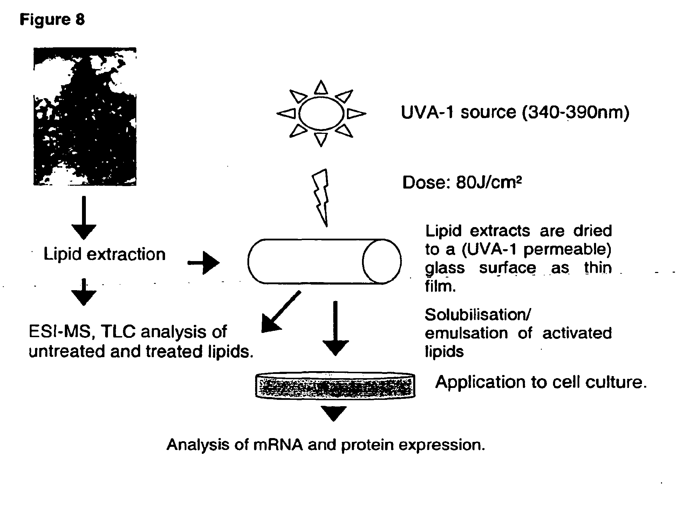

An extract from organic material is treated with UV light as follows (Fig. 8): the extract is applied as a thin film to a glass support that is permeable for light with wavelengths longer than 340 nm. The fixation of the extract to the carrier is performed by high speed agitation of the lipids which are solubilized in chloroform during gassing with an inert gas like nitrogen or argon. The glass support, preferably a glass cylinder that can be airtightly sealed, is then irradiated with UVA-1 (wavelength 340 to 400 nm) until a fluency of 80J/cm² is reached, measured at the basal point of the glass support. As a light source for UVA-1 a Mutzhas Supersun 5000-type solar simulator (Mutzhas, Munich, Germany) filtered for the emission of UVA-1 (340-390 nm) is used.

Subsequently, the substances are agitated at high speed with chloroform or a non-organic solvent (phosphate buffered saline, water, medium) to solubilize, emulgate or suspend them. In order to obtain a solution, emulgation or suspension containing said substances in a concentration for example of 100 µg/ml, said substances are weighed after irradiation and then solubilized, emulgated or suspended as described above.

Both the extract obtained after isopropanol extraction and the extract obtained after chloroform/methanol purification can be treated with UV light. While both kinds of extracts do not dissolve completely before UV treatment, either of them can be easily solubilised after UV treatment.

### Thin layer chromatography of UVA-1 treated Physcomitrella patens extracts

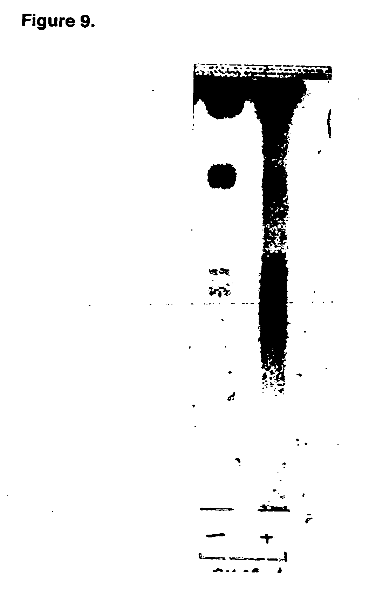

Figure 9 shows a thin layer chromatography of UVA-1 treated (right column) and untreated (left column) *Physcomitrella patens* extracts that were used in experiments on induction of HO-1 expression wherein they exhibited an activity to induce HO-1 expression in keratinocytes. Thin layer chromatography was performed as described in Example 1. Equal amounts of lipids were loaded at the bottom line.

By capillary forces the lipids migrate upward. The more polar a lipid is the shorter it will migrate upwards. The polarity depends
(a) on the charge of the molecule (Phosphatid acid with 2 negative charges migrates slower)
(b) length of the aliphatic (hydrophobic) fatty acid chains (molecules with longer chains migrate slower since they are less polar)
(c) number of oxygen containing groups (the higher the oxygen content is the higher is the polarity and the shorter is the migration distance).
The thin layer chromatography clearly shows an increase of oxidized fatty acids after UVA-1 treatment of *Physcomitrella patens* extracts.

### Mass spectrometry of a crude lipid extract from Physcomitrella patens

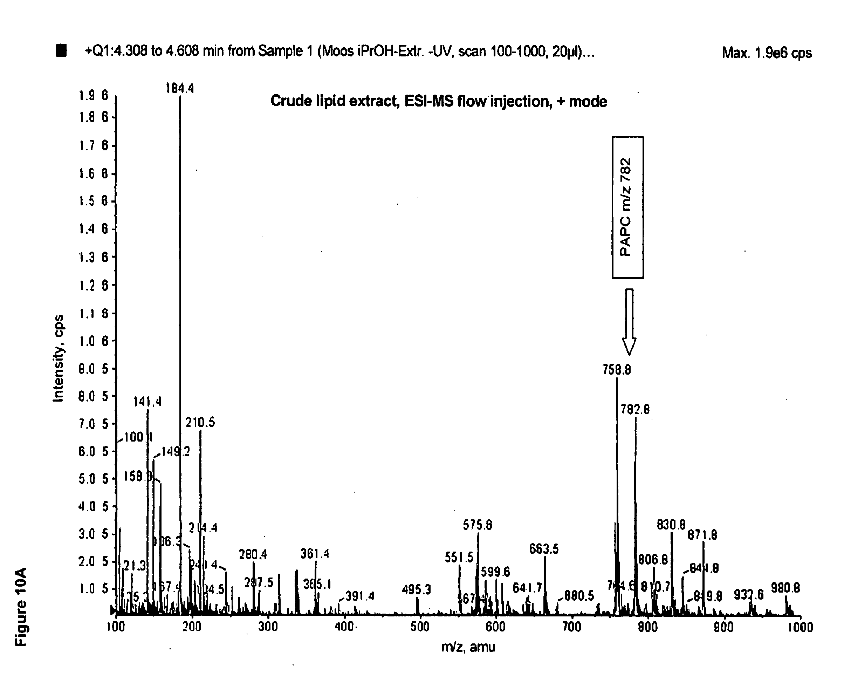

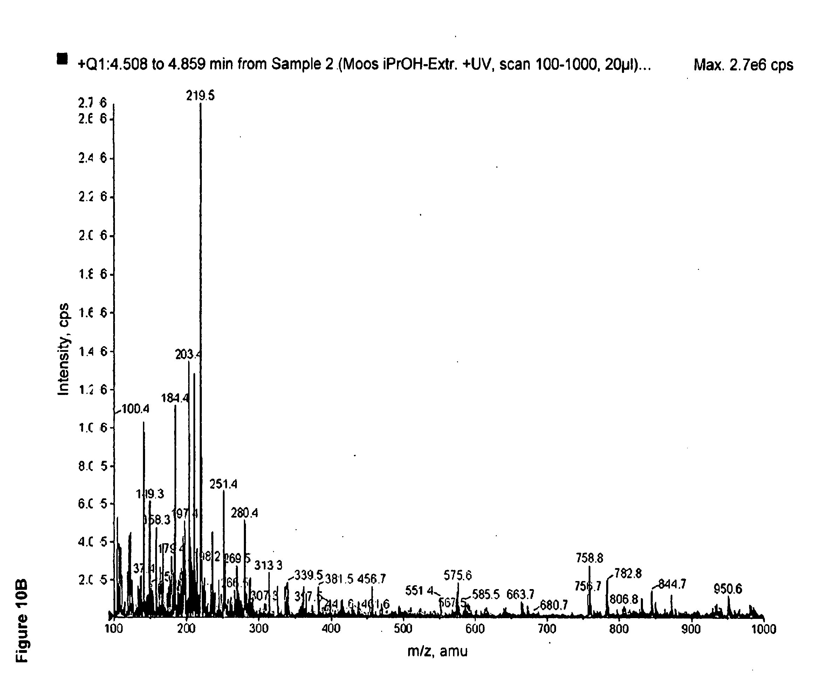

Mass spectrometry of an UVA-1 treated extract from *Physcomitrella patens* was performed as described in Example 1. After treatment with UVA-1 the relative abundance of PAPC is clearly reduced as compared to an untreated extract (Fig. 10). Since the extract contains a large number of lipids and other compounds the small amount of oxidation products of PAPC cannot be determined by mass spectrometry. However, one can assume that the same oxidation products of PAPC arise as can be observed by mass spectrometry after UVA-1 treatment of PAPC (Fig. 3).

### Example 5:

### Expression of Heme oxygenase (HO-1) is induced by an activated extract derived from Physcomitrella patens but not by extracts derived from corn, olive and cucurbits which comprise no or to a very low extent (i.e. merely 2 double bonds) polyunsaturated fatty acids even in the presence of a sensitizer (Rose Bengal)

### Materials and Methods

### Cells and Lipids

Cells and lipids are used as described in example 2.

### Commercial oils

Commercial oils: Corn oil [oil from *Zea mays* (Mazola, Austria)], olive oil (Spar, Austria), pumpkin seed oil [oil derived from seeds of *Cucurbita pepo L. var. styriaca I.* (Spar, Austria)]

### Extract from Physcomitrella patens

Total lipid extract derived from *Physcomitrella patens* extract from *Physcomitrella patens* is obtained using a modification of Nichols' method of lipid extraction as described in Example 4.

### UV-irradiation

Treatment of the lipids or extract with UVA-1 is performed as described in Example 4

### Treatment of fibroblasts with UV-irradiated extract

Fibroblasts are treated with lipids or extract as described in Example 1 relating to treatment with oxPAPC. Total RNA is isolated 4h after stimulation with 100 µg/ml of lipids or extract.

### Quantitative real-time PCR (qPCR).

Relative expression of HO-1 mRNA in dermal fibroblasts is determined 4h after stimulation with lipids or extracts as specified above using quantitative real-time PCR (qPCR). Normalization is standardized by comparison to the expression of the housekeeping gene beta-2-microglobulin (B2M) as described in Example 1.

### Technical details and results

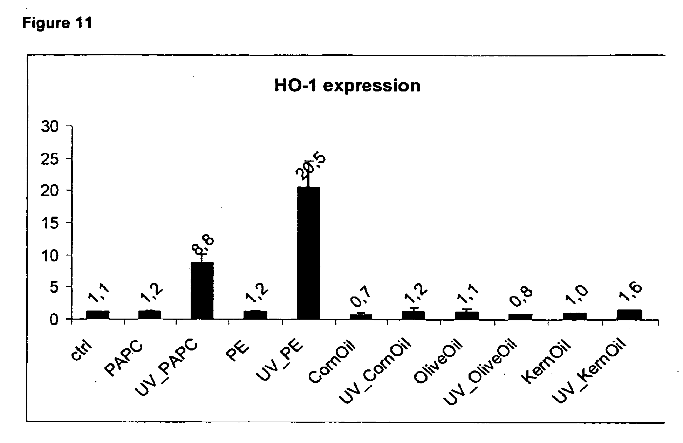

In order to test whether Heme Oxygenase 1 (HO-1) inducing ability is conferred to lipids that contain groups derived from PUFA (polyunsaturated fatty acids) by UVA-1 irradiation treatment lipids and lipid extracts were treated with UVA-1 (80J/cm²). The treated and untreated lipids (100µg/ml each) were added to cultured dermal fibroblasts and relative HO-1 expression was determined using real time PCR (Fig. 11). The lipids used were the pure lipid PAPC and the following "lipid extracts" (commercially available plant oils) Corn Oil, Olive Oil, and C.pepo seed oil (Kern Oil) and the exemplified inventive *Physcomitrella patens* lipid extract (PE). UVA-1 treated *Physcomitrella patens* extracts and to a lesser extent UVA-1 treated PAPC (UV_PAPC) but not untreated *Physcomitrella patens* extracts (PE) or untreated PAPC (PAPC) induce Heme Oxygenase 1 (HO-1) mRNA expression in dermal fibroblasts 4h after treatment with 100 µg/ml of lipid or extract relative to control levels (ctrl). Olive oil, UVA-1 treated olive oil, pumpkin seed oil, UVA-1 treated pumpkin seed oil, corn oil or UVA-1 treated corn oil were not able to increase Heme Oxygenase mRNA expression.

While the treatment of pure PAPC and *Physcomitrella patens* extracts with UVA-1 resulted in an capability of the treated lipid or extract to induce HO-1 mRNA expression, the other lipids were not activated by a corresponding treatment with UVA-1. Accordingly, as laid down in the present invention, *Physcomitrella patens* extracts comprise compounds that can be activated by irradiation with UVA-1 and said activated *Physcomitrella patens* extracts have beneficial effects in terms of stress response, as documented by the upregulation of HO-1 in the here provided cellular systems.

In order to test whether the presence of a sensitizer (such as Rose Bengal described herein above in Example 1) might facilitate activation of lipids by treatment with UVA-1, commercially available olive oil was mixed with Rose Bengal and subject to UVA-1 treatment (10J/cm²) in the presence of 90 mM Rose Bengal ("RB-Olive Oil'"). As a control olive oil was treated with UVA-1 alone (40 J/cm², "UV-Olive Oil") in the absence of Rose Bengal.

Four hours after UVA-1 treatment total RNA was extracted and the relative expression of Heme Oxygenase 1 was measured using real time PCR. As shown in figure 12, UVA-1 treated PAPC (UV_PAPC) and PAPC treated in the presence of Rose Bengal (90 µM, "RB-PAPC") but not untreated PAPC (PAPC) induce Heme Oxygenase 1 (HO-1) mRNA expression in dermal fibroblasts 4h after treatment with 100 µg/ml of lipid relative to control levels (ctrl). Olive oil, UVA-1 treated olive oil and olive oil UVA-1 treated in the presence of RB were not able to increase Heme Oxygenase mRNA expression. These findings are in line with our previous notion that Corn Oil, Olive Oil, and C.pepo seed oil (Kern Oil) oils cannot be activated according to the method of the present invention. Even the presence of the sensitizer RB did not lead to an activation of such oils.

In the present invention (Example 1), it has been shown that addition of Rose Bengal as a singlet oxygen generating compound facilitates formation of activated and HO-1 inducing PAPC even at lower fluencies. In the presence of Rose Bengal a fluency of 10J/cm² is sufficient to activate PAPC which in turn will induce a relative expression of HO-1 up to a plateau (i.e. maximum) level. In the absence of Rose Bengal a four fold higher fluency (40 J/cm²) is needed to induce a comparable, relative expression up to a plateau level. In other words, PAPC treated with UVA-1 alone induces a relative expression of HO-1 up to a plateau level. It is therefore assumed that the relative expression of HO-1 in the presence of Rose Bengal is not increased. This might be one reason why addition of RB did not lead to a pronounced of relative expression of HO-1. As shown herein below in Example 8, addition of RB to fish oil led to a remarkable increase in the relative expression of HO-1 in cells treated with activated fish oil. Fish oil treated with UVA-1 only might not be sufficient to induce a relative expression of HO-1 up to a plateau level, i.e. UVA-1 treatment of fish oil in the presence of RB leads to an increase of relative expression of HO-1.

Without being bound by theory, the capability of an extract/lipid of being activated according to the present method might be influenced by the presence of at least one (poly)unsaturated fatty acid, group derived therefrom or a lipid comprising such a group, preferably a PUFA or group derived therefrom comprising at least 4 double bonds, in the extract as described herein, supra; i.e. the higher the content of at least one group derived from a (poly)unsaturated fatty acid in an extract or a lipid comprising such a group the higher the capability of the extract of being activated may be. In this context a preferred PUFA or group derived therefrom comprises at least 4 double bonds and/or comprises at least 20 C atoms. Most preferably, said group is derived from arachidonic acid and a preferred phospholipid comprising such a group is PAPC. It is known that olive oil contains predominantly oleic acid (18:1), and smaller amounts of linoleic (18:2) and palmitic (16:0) acid (Milosevic et al International Journal of Food Science and Technology 2002, 37, 523-526). Thus, one reason for the incapability of olive oil of being activated by UVA-1 treatment may be the absence or low content of at least one group derived from a polyunsaturated fatty acid comprising at least 4 double bonds described herein above.

A sensitizer such as Rose Bengal employed in accordance with the method of the present invention may lead to an enhanced activation of the extract. Also the presence of "natural" sensitizers such as chlorophyll in an extract as described herein above may contribute to an enhanced activation of the extract upon UVA-1 treatment. Also here, the higher the content of at least one sensitizer the higher the capability of the extract of being activated may be. However, olive oil could not be activated even in the presence of a sensitizer (Rose Bengal).

### Example 6:

### Expression of Heme oxygenase (HO-1) and Glutathione Cysteine Ligase Modifier subunit (GCLM) induced by an UVA-1 treated extract derived from the moss Physcomitrella patens is stronger relative to non-treated control cells, cells treated with autoxidized PAPC or untreated extract

### Materials and Methods

Cells are used as described in example 1. PAPC is used as described in example 2. An extract from *Physcomitrella patens* is obtained as described in example 4. Autoxidized PAPC (oxPAPC) is obtained according to the method described in example 1. UV irradiation of the lipid or extract is performed as described in example 4.

Treatment of cells (keratinocytes, dermal fibroblasts, skin equivalents) with UV-irradiated extract or lipid is performed as described in example 5. Relative expression of HO-1 mRNA or GCLM mRNA in cells is determined 4h after stimulation with lipids or extracts using quantitative real-time PCR (qPCR) as described in example 1. Normalization is standardized by comparison to the expression of the housekeeping gene beta-2-microglobulin (B2M) as described in example 1. The following primers are used for qPCR of GCLM:
fwd: gcgaggagcttcatgattgt; rev: tgtgcaactccaaggactga. Immunofluorescence, immunohistochemistry, thin-layer chromatography and mass spectrometry are performed as described in example 1.

### Results

As illustrated in the appended figures UVA-1 treated extract from *Physcomitrella patens* induces Heme Oxygenase 1 (HO-1) mRNA expression in skin cells (keratinocytes, dermal fibroblasts) to a much larger extent than in non-treated control cells or cells treated with non-activating (non UVA-1 treated) *Physcomitrella patens* extracts (Fig. 13., Fig. 14) or cells treated with autoxidized PAPC (Fig. 15, Fig. 16). Correspondingly, UVA-1 treated extract from *Physcomitrella patens* induces HO-1 protein expression in skin equivalents to a much larger extent than in non-treated control skin equivalents or skin equivalents treated with autoxidized PAPC (Fig. 17). Glutathione Cysteine Ligase Modifier subunit (GCLM) mRNA expression induced in primary dermal fibroblasts by an UVA-1 treated extract from *Physcomitrella patens* was also much stronger as compared to control levels or the untreated extract (Fig. 18). GCLM is a rate limiting enzyme for glutathione (GSH) synthesis.

Glutathione (GSH) levels showed a slight increase after induction with UV-treated but not untreated extract in dermal fibroblasts 18 hours after treatment relative to control levels (Fig. 19). UV treated PAPC leads to a stronger increase of GSH levels as compared to UV-treated extract (Fig. 20). One may assume that various compounds of the extract might contribute to the induction of HO-1 mRNA expression in an accumulative manner resulting in a higher mRNA expression induced by the extract relative to UV treated PAPC. In contrast, PEIPC might be the major or sole effector of the induction of GCLM mRNA expression and subsequent Glutathione synthesis. Since PEIPC may be relatively more abundant in UVA-1 treated PAPC compared to UVA-1 treated extract one may assume that this fact may explain the relative weak increase of GSH levels in dermal fibroblasts after treatment with activated extract. However, it is to be stressed that the activated extract is a strong effector of the antioxidant response in general though in some cases it might not be able to induce genes related to that antioxidant response as efficiently as pure UV treated PAPC.

To summarize, it was surprisingly found that a UVA-1 treated extract from *Physcomitrella patens* induces the mRNA expression of two genes, namely Heme Oxygenase 1 and Glutathione Cysteine Ligase Modifier subunit (GCLM), which play an important role in the antioxidant response. Furthermore, the upregulation of Heme Oxygenase 1 protein expression was confirmed in skin equivalents. The upregualation of GCLM led to an increased synthesis of Glutathione which is known as an important antioxidant cellular compound and accordingly to increased Glutathione levels. Unexpectedly, the UVA-1 treated extract from *Physcomitrella patens* induced the upregulation of HO-1 and GCLM to a much larger extent than autoxidized PAPC. UVA-1 treated PAPC was not able to induce a higher HO-1 mRNA expression in skin cells than autoxidized PAPC which means that PAPC is not activated to a larger extent by the UVA-1 treatment. Thus it is clearly shown that the UVA-1 treated extract from *Physcomitrella patens* is a much stronger effector of the antioxidant response than pure PAPC.

### Example 7:

### Expression of Heme oxygenase (HO-1) induced by an UVA-1 treated extract derived from the fern (Thelypteris noveboracensis) is stronger relative to non-treated control cells

An extract from the fern (*Thelypteris noveboracensis*) is obtained as described in example 4. Wild growing "New York Fern" (*Thelypteris noveboracensis*) was harvested in central Virginia, USA. Lipids were extracted as above described for P.patens. UV irradiation of the extract is performed as described in example 4. Treatment of cells (dermal fibroblasts) with UV-irradiated extract is performed as described in example 5. Relative expression of HO-1 mRNA in cells is determined 4h after stimulation extracts using quantitative real-time PCR (qPCR) as described in example 1.

As shown in Figure 21, UVA-1 irradiated fern extract surprisingly induced a strong heme oxygenase expression. Thus it is shown that not only the UVA-1 treated extract from the moss *Physcomitrella patens* but also from a further plant, namely the fern *Thelypteris noveboracensis,* is capable of inducing a strong antioxidant stress response. It was demonstrated herein for the first time that extracts derived from ferns activated in accordance with the present invention are capable of inducing an antioxidant stress response.

### Example 8:

### Expression of Heme oxygenase (HO-1) induced by an UVA-1 treated extract derived from fish (fish oil) is stronger relative to non-treated control cells or untreated extract

Fish oil employed herein is contained in "Harris Teeter Naturals Fish oil concentrate capsules" distributed by Harris Teeter Matthews, NC 28105. According to the manufacturer, 1g Fish oil contains 180mg eicosapentaenoic acid (EPA) and 120mg docosahexaenoic acid (DHA). UV irradiation of the fish oil is performed as described in example 4.

Treatment of cells (dermal fibroblasts) with UV-irradiated extract or lipid is performed as described in example 5. Relative expression of HO-1 mRNA in cells is determined 4h after stimulation with lipids or extracts using quantitative real-time PCR (qPCR) as described in example 1 with the exception that fish oil in a concentration of 5000 µg/ml is used.

To test whether an extract derived from fish treated according to the method of the invention may also be capable of inducing an antioxidant stress response, we treated dermal fibroblasts with 5000 µg/ml of fish oil irradiated with 40 J/cm² UVA-1 ("Fish Oil UVA-1") and with 10J/cm² UVA-1 in the presence of 90 µM Rose Bengal ("Fish Oil UVA-1 RB").

It is demonstrated herein for the first time that fish oil can be activated by UVA-1 treatment in accordance with the present invention (Fig. 22). Further, it is shown herein that the presence of a sensitizer (RoseBengal) even increases the capability of the activated extract of inducing an antioxidant stress response.

In sum, the examples provided herein above clearly show that extracts derived from non-mammalian cells or non-mammalian organisms which are capable of synthesizing (poly)unsaturated fatty acids, preferably a PUFA or group derived therefrom comprising at least 4 double bonds, (such as mosses, ferns and fish) can successfully be activated according to the present method. The activated extracts are unexpectedly capable of inducing a strong antioxidant response. Surprisingly, it was also shown that extracts to be employed herein are capable of inducing an even increased antioxidant stress response in the presence of a sensitizer (e.g. Rose Bengal). In contrast thereto, olive oil and other commercially available oils (commonly known as containing a high content of unsaturated fatty acids) treated in accordance with the present invention and even in the presence of a sensitizer were not capable of inducing such a response.

## Claims

1. A method for producing an extract capable of inducing an antioxidant stress response comprising the step of treating an extract from organic material with UV-A1 light, wherein a fluency of at least 1 J/cm² to 200 J/cm² of said UV light is applied, wherein said extract from organic material is derived from a bryophyte or algae capable of synthesizing polyunsaturated fatty acids comprising at least 4 double bonds and at least 20 C-atoms, and
wherein said extract from organic material is a lipid extract comprising at least 0.1 (v/v) % and at the utmost 50 (v/v) % 1-palmitoyl-2-arachidonyl-sn-glycero-3-phosphorylcholine (PAPC).

2. The method according to claim 1, wherein said polyunsaturated fatty acid is a fatty acid group of a lipid.

3. The method according to claim 1 or 2, wherein said bryophyte is selected from the group consisting of liverworts (Marchantiophyta), hornworts (Anthoceratophyta) and mosses (Bryophyta).

4. The method according to claim 3, wherein said bryophyte (moss) belongs to the genus *Physcomitrella.*

5. The method according to claim 4, wherein said moss is *Physcomitrella patens.*

6. The method according to any one of claims 1 to 5, wherein said UVA-1 has a wavelength from 315 to 400 nm.

7. The method according to any one of claims 1 to 6, wherein said UV-A1 has a wave length from 340 to 390 nm.

8. The method according to any one of claims 1 to 7, wherein a fluency of 5 J/cm² to 100 J/cm² or at least 10 J/cm² to 80 J/cm² of said UV light is applied.

9. The method according to any one of claims 1 to 8, wherein a fluency of 80 J/cm² of said UV light is applied.

10. The method according to any one of claims 1 to 9, wherein said polyunsaturated fatty acid or group derived therefrom is or is derived from arachidonic acid, docosahexaenoic acid (DHA), eicosapentaenoic acid (EPA) or docosapentaenoic acid (DPA).

11. The method according to any one of claims 1 to 10, wherein said extract capable of inducing an antioxidant stress response is a lipid extract.

12. The method according to claim 11, wherein said lipid extract comprises an oxidized polyunsaturated fatty acid having an epoxyisoprostanoid group.

13. The method according to claim 12, wherein said epoxyisoprostanoid group is an epoxyisoprostanoid E2 group.

14. The method according to claim 12 or 13, wherein said oxidized lipid is 1-palmitoyl-2-(epoxyisoprostane E2)-*sn*-glycero-3-phosphorylcholine (PEIPC).

15. An extract obtainable by the method of any one of claims 1 to 14.

16. An extract obtainable by the method of any one of claims 1 to 14 for the preparation of a cosmetic composition.

17. A non-medical use of an extract obtainable by the method of any one of claims 1 to 14 for the cosmetic treatment, amelioration or prevention of symptoms of ageing which are related to oxidative stress.

18. The non-medical use of claim 17, wherein said symptom of ageing is selected from the group consisting of skin ageing, wrinkle formation and saggy skin.

19. A pharmaceutical composition comprising an extract obtainable by the method of any one of claims 1 to 14 for use in treating skin cancer, acute inflammatory diseases, graft-versus-host disease, skin disorders due to exposure to UV radiation and for repairing or combating skin ageing, whether photoinduced or chronological, any pathologies associated with chronological or actinic ageing, preferably xerosis, dermatological disorders, preferably common acne, comedone-type acne, polymorphic acne, acne rosacea, nodulocystic acne, acne conglobata, senile acne, secondary acne such as solar acne, acne medicamentosa or occupational acne, keratinization disorders, preferably ichthyosis, or ichthyosiform states, dermatological conditions with an inflammatory immunoallergic component, preferably psoriasis, atopic dermatitis, whether cutaneous, mucosal or ungular cutaneous atopy, such as eczema or respiratory atopy or gingival hypertrophy, dermal or epidermal proliferations whether benign or malignant, whether of viral or non-viral origin, preferably verruca vulgaris, verruca plana, condyloma and epidermodysplasia verruciformis, dermatological disorders such as immune dermatoses, preferably lupus erythematosus, bullous immune diseases and collagen diseases, such as scleroderma.

## Patentansprüche

1. Verfahren zur Herstellung eines Extraktes, das fähig ist, eine Antioxidans-Stressantwort zu induzieren, umfassend den Schritt des Behandelns eines Extraktes aus organischem Material mit UV-A1-Licht,
wobei eine Fluenz von mindestens 1 J/cm² bis 200 J/cm² des UV-Lichts angewendet wird, wobei der Extrakt aus organischem Material aus einer Moospflanze oder einer Alge abgeleitet wurde, die fähig ist, mehrfach ungesättigte Fettsäuren zu synthetisieren, die mindestens 4 Doppelbindungen und mindestens 20 C-Atomen umfassen, und
wobei der Extrakt aus organischem Material ein Lipid-Extrakt ist, der mindestens 0,1 (v/v) %, und höchstens 50 (v/v) % 1-Palmitoyl-2-Arachidonyl-sn-glycero-3-phosphorylcholin (PAPC) umfasst.

2. Verfahren nach Anspruch 1, wobei die mehrfach ungesättigte Fettsäure eine Fettsäure-Gruppe eines Lipids ist.

3. Verfahren nach Anspruch 1 oder 2, wobei die Moospflanze ausgewählt ist aus der Gruppe bestehend aus Lebermoosen (Marchantiophyta), Hornmoosen (Anthoceratophyta) und Laubmoosen (Bryophyta).

4. Verfahren nach Anspruch 3, wobei die Moospflanze (Laubmoos) zur Gattung *Physcomitrella* gehört.

5. Verfahren nach Anspruch 4, wobei das Laubmoos *Physcomitrella patens* ist.

6. Verfahren nach einem der Ansprüche 1 bis 5, wobei das UVA-1 eine Wellenlänge von 315 bis 400 nm hat.

7. Verfahren nach einem der Ansprüche 1 bis 6, wobei das UV-A1 eine Wellenlänge von 340 bis 390 nm hat.

8. Verfahren nach einem der Ansprüche 1 bis 7, wobei eine Fluenz von 5 J/cm² bis 100 J/cm² oder mindestens 10 J/cm² bis 80 J/cm² des UV-Lichts angewendet wird.

9. Verfahren nach einem der Ansprüche 1 bis 8, wobei eine Fluenz von 80 J/cm² des UV-Lichts angewendet wird.

10. Verfahren nach einem der Ansprüche 1 bis 9, wobei die mehrfach ungesättigte Fettsäure oder die davon abgeleitete Gruppe Arachidonsäure, Docosahexaensäure (DHA), Eicosapentaensäure (EPA) oder Docosapentaensäure (DPA) ist oder davon abgeleitet ist.

11. Verfahren nach einem der Ansprüche 1 bis 10, wobei der Extrakt, der fähig ist, eine Antioxidans-Stressantwort zu induzieren, ein Lipid-Extrakt ist.

12. Verfahren nach Anspruch 11, wobei der Lipid-Extrakt eine oxidierte mehrfach ungesättigte Fettsäure mit einer Epoxyisoprostanoid-Gruppe umfasst.

13. Verfahren nach Anspruch 12, wobei die Epoxyisoprostanoid-Gruppe eine Epoxyisoprostanoid-E2-Gruppe ist.

14. Verfahren nach Anspruch 12 oder 13, wobei das oxidierte Lipid 1-Palmitoyl-2-(epoxyisoprostan E2)-*sn-*glycero-3-phosphorylcholin (PEIPC) ist.

15. Extrakt, erhältlich durch das Verfahren nach einem der Ansprüche 1 bis 14.

16. Extrakt, erhältlich durch das Verfahren nach einem der Ansprüche 1 bis 14 zur Herstellung einer kosmetischen Zusammensetzung.

17. Nicht-medizinische Verwendung eines Extrakts, der erhältlich ist durch das Verfahren nach einem der Ansprüche 1 bis 14, zur kosmetischen Behandlung, Linderung oder Vorbeugung von Symptomen der Alterung, die mit oxidativem Stress verbunden sind.

18. Nicht-medizinische Verwendung nach Anspruch 17, wobei das Symptom der Alterung ausgewählt ist aus der Gruppe bestehend aus Hautalterung, Faltenbildung und schlaffe Haut.

19. Arzneimittel umfassend einen Extrakt, der erhältlich ist durch das Verfahren nach einem der Ansprüche 1 bis 14, zur Verwendung bei der Behandlung von Hautkrebs, akute Entzündungserkrankungen, Graft-versus-Host-Krankheit, Hauterkrankungen aufgrund der Aussetzung von UV-Strahlung und für die Reparatur oder Bekämpfung von Hautalterung, die lichtinduziert oder chronologisch ist, jede beliebige Pathologie, die mit chronologischer oder aktinischer Alterung assoziiert sind, vorzugsweise Xerose, dermatologischen Störungen, vorzugsweise Akne vulgaris, Mitesser-Akne-Typ, polymorphe Akne, Acne rosacea, Nodulozystische Akne, Akne conglobata, senile Akne, sekundäre Akne wie Acne solaris, Acne medicamentosa oder Akne als Berufsdermatose, Verhornungsstörungen, vorzugsweise Ichthyosis oder ichthyosiforme Zustände, dermatologischen Zustände mit einer entzündlichen immunoallergischen Komponente, vorzugsweise Psoriasis, atopische Dermatitis, entweder der Haut, der Schleimhaut oder kutane Atopie des Nagels, wie Ekzeme oder Respiratorische Atopie oder Hypertrophie des Zahnfleisches, dermalen oder epidermalen Proliferationen, die gutartig oder bösartig, viralen oder nicht-viralen Ursprungs sind, vorzugsweise Verruca vulgaris, Verruca plana, Kondylom und Epidermodysplasia verruciformis, dermatologischen Störungen, wie Immundermatosen, vorzugsweise Lupus erythematodes, bullösen Immunerkrankungen und Kollagenkrankheiten, wie Sklerodermie.

## Revendications

1. Procédé de production d'un extrait capable d'induire une réponse à un stress oxydant comprenant l'étape consistant à traiter un extrait d'une matière organique avec une lumière UV-A1, dans lequel une fluence d'au moins 1 J/cm² à 200 J/cm² de ladite lumière UV est appliquée, dans lequel ledit extrait d'une matière organique est dérivé d'un bryophyte ou d'algues capables de synthétiser des acides gras polyinsaturés comprenant au moins 4 doubles liaisons et au moins 20 atomes de C, et
dans lequel ledit extrait d'une matière organique est un extrait lipidique comprenant au moins 0,1 % v/v et au plus 50 % v/v de 1-palmitoyl-2-arachidonyl-sn-glycéro-3-phosphorylcholine (PAPC).

2. Procédé selon la revendication 1, dans lequel ledit acide gras polyinsaturé est un groupe acide gras d'un lipide.

3. Procédé selon les revendications 1 ou 2, dans lequel ledit bryophyte est choisi dans le groupe constitué des hépatiques (Marchantiophyta), des anthocérotes (Anthoceratophyta) et des mousses (Bryophyta).

4. Procédé selon la revendication 3, dans lequel ledit bryophyte (mousse) appartient au genre *Physcomitrella.*

5. Procédé selon la revendication 4, dans lequel ladite mousse est *Physcomitrella patens.*

6. Procédé selon l'une quelconque des revendications 1 à 5, dans lequel ledit UVA-1 a une longueur d'onde de 315 à 400 nm.

7. Procédé selon l'une quelconque des revendications 1 à 6, dans lequel ledit UV-A1 a une longueur d'onde de 340 à 390 nm.

8. Procédé selon l'une quelconque des revendications 1 à 7, dans lequel une fluence de 5 J/cm² à 100 J/cm² ou d'au moins 10 J/cm² à 80 J/cm² de ladite lumière UV est appliquée.

9. Procédé selon l'une quelconque des revendications 1 à 8, dans lequel une fluence de 80 J/cm² de ladite lumière UV est appliquée.

10. Procédé selon l'une quelconque des revendications 1 à 9, dans lequel ledit acide gras polyinsaturé ou groupe dérivé de celui-ci est de l'acide arachidonique, de l'acide docosahexaénoïque (DHA), de l'acide éicosapentaénoïque (EPA) ou de l'acide docosapentaénoïque (DPA) ou est dérivé de ceux-ci.

11. Procédé selon l'une quelconque des revendications 1 à 10, dans lequel ledit extrait capable d'induire une réponse à un stress oxydant est un extrait lipidique.

12. Procédé selon la revendication 11, dans lequel ledit extrait lipidique comprend un acide gras polyinsaturé oxydé comportant un groupe époxyisoprostanoïde.

13. Procédé selon la revendication 12, dans lequel ledit groupe époxyisoprostanoïde est un groupe époxyisoprostanoïde E2.

14. Procédé selon la revendication 12 ou 13, dans lequel ledit lipide oxydé est la 1-palmitoyl-2-(époxyisoprostane E2)-*sn*-glycéro-3-phosphorylcholine (PEIPC).

15. Extrait pouvant être obtenu par le procédé selon l'une quelconque des revendications 1 à 14.

16. Extrait pouvant être obtenu par le procédé selon l'une quelconque des revendications 1 à 14 pour la préparation d'une composition cosmétique.

17. Utilisation non médicale d'un extrait pouvant être obtenu par le procédé selon l'une quelconque des revendications 1 à 14 pour le traitement cosmétique, l'amélioration ou la prévention des symptômes du vieillissement qui sont liés au stress oxydatif.

18. Utilisation non médicale selon la revendication 17, dans laquelle ledit symptôme du vieillissement est choisi dans le groupe constitué du vieillissement de la peau, de la formation de rides et de l'affaissement de la peau.

19. Composition pharmaceutique comprenant un extrait pouvant être obtenu par le procédé selon l'une quelconque des revendications 1 à 14 pour l'utilisation dans le traitement du cancer de la peau, des maladies inflammatoires aiguës, de la réaction de greffe contre hôte, des troubles cutanés dus à l'exposition aux rayonnements UV, et pour la réparation ou la lutte contre le vieillissement de la peau, qu'il soit photoinduit ou chronologique, toutes pathologies associées à un vieillissement chronologique ou actinique, de préférence le xérosis, les troubles dermatologiques, de préférence l'acné vulgaire, l'acné comédonienne, l'acné polymorphique, l'acné rosacée, l'acné nodulokystique, l'acné conglobata, l'acné sénile, l'acné secondaire telle que l'acné solaire, l'acné d'origine médicamenteuse ou l'acné professionnelle, les troubles de la kératinisation, de préférence l'ichtyose, ou les états ichtyosiformes, les affections dermatologiques associées à une composante immunoallergique inflammatoire, de préférence le psoriasis, la dermatite atopique, qu'il s'agisse d'une atopie cutanée, des muqueuses ou cutanée unguéale, telle que l'eczéma ou l'atopie respiratoire ou l'hypertrophie gingivale, les proliférations dermiques ou épidermiques, qu'elles soient bénignes ou malignes, qu'elles soient d'origine virale ou non virale, de préférence la verrue vulgaire, la verrue plane, le condylome et l'épidermodysplasie verruciforme, les troubles dermatologiques tels que les dermatoses immunitaires, de préférence le lupus érythémateux, les maladies immunitaires bulleuses et les maladies du collagène, telles que la sclérodermie.


### SEQUENCE LISTING

<110> Medizinische Universität Wien
<120> A process to activate extracts derived from living organisms using long wave UV irradiation and compositions comprising said activated extract
<130> N1324 PCT S3
<160> 6
<170> PatentIn version 3.3
<210> 1
   <211> 24
   <212> DNA
   <213> Artificial
<220>
   <221> source
   <223> /note="description of artificial sequence:primer"
<400> 1
   aagattgccc agaaagccct ggac 24
<210> 2
   <211> 24
   <212> DNA
   <213> Artificial
<220>
   <221> source
   <223> /note="description of artificial sequence:primer"
<400> 2
   aactgtcgcc accagaaagc tgag 24
<210> 3
   <211> 21
   <212> DNA
   <213> Artificial
<220>
   <221> source
   <223> /note="description of artificial sequence:primer"
<400> 3
   gatgagtatg cctgccgtgt g 21
<210> 4
   <211> 19
   <212> DNA
   <213> Artificial
<220>
   <221> source
   <223> /note="description of artificial sequence:primer"
<400> 4
   caatccaaat gcggcatct 19
<210> 5
   <211> 20
   <212> DNA<213> Artificial
   <213> Artificial
<220>
   <221> source
   <223> /note="description of artificial sequence:primer"
<400> 5
   gcgaggagct tcatgattgt 20
<210> 6
   <211> 20
   <212> DNA
   <213> Artificial
<220>
   <221> source
   <223> /note="description of artificial sequence:primer"
<400> 6
   tgtgcaactc caaggactga 20